# EUROPEAN PATENT SPECIFICATION

(11) **EP 3 916 111 B1**
(45) Date of publication and mention of the grant of the patent: **09.08.2023**
(21) Application number: 20744348.2
(22) Date of filing: 17.01.2020
(51) Int. Cl.: C12Q 1/70

(54) **GENERAL-PURPOSE PRIMER SETS FOR DETECTING FLAVIVIRUSES AND USE THEREOF**
MEHRZWECK-PRIMERSETS ZUM NACHWEIS VON FLAVIVIREN UND IHRE VERWENDUNG
ENSEMBLES D'AMORCES UNIVERSELLES POUR LA DÉTECTION DE FLAVIVIRUS ET LEUR UTILISATION

(30) Priority: 25.01.2019 KR 20190009813
(43) Date of publication of application: 01.12.2021
(73) Proprietor: Industry Foundation of Chonnam National University, Gwangju 61186 (KR)
(72) Inventor: HAN, Yeon, Soo, Gwangju 61037 (KR); PARK, Ki, Beom, Gwangju 62343 (KR); JO, Yong, Hun, Gwangju 61220 (KR); BAE, Young, Min, Jeongeup-si, Jeollabuk-do 56105 (KR)
(74) Representative: Plougmann Vingtoft a/s
(86) International application number: PCT/KR2020/000886
(87) International publication number: WO 2020/153673

(56) References cited:
- WO-A1-2018/029452
- KR-A- 20070 121 853
- KR-A- 20120 086 028
- KR-A- 20180 098 778
- KR-B1- 100 942 388
- VINA-RODRIGUEZ ARIEL ET AL: "A Novel Pan- Flavivirus Detection and Identification Assay Based on RT-qPCR and Microarray", BIOMED RESEARCH INTERNATIONAL, vol. 2017, 1 January 2017 (2017-01-01), pages 1-12, XP055888262, ISSN: 2314-6133, DOI: 10.1155/2017/4248756 Retrieved from the Internet: URL:https://downloads.hindawi.com/journals /bmri/2017/4248756.pdf>
- MAHER-STURGESS SHERYL L ET AL: "Universal primers that amplify RNA from all three flavivirus subgroups", VIROLOGY JOURNAL, BIOMED CENTRAL, LONDON, GB, vol. 5, no. 1, 24 January 2008 (2008-01-24), page 16, XP021038316, ISSN: 1743-422X
- SCARAMOZZINO N ET AL: "Comparison of flavivirus universal primer pairs an developmenof an rapid, highly sentitive heminested reverse transcription-PCR assay for detection of flaviviruses targeted to an conserved region of the NS5 gene sequences", JOURNAL OF CLINICAL MICROBIOLOGY, AMERICAN SOCIETY FOR MICROBIOLOGY, US, vol. 39, no. 5, 1 May 2001 (2001-05-01), pages 1922-1927, XP002988841, ISSN: 0095-1137, DOI: 10.1128/JCM.39.5.1922-1927.2001

## Description

### Technical Field

The present invention was made with the support of the Ministry of Health and Welfare, Republic of Korea, under Project No. HG18C0028, which was executed in the research project named "Proposal of standardization methods for efficient surveillance of infectious disease vectors (hard ticks and mosquitoes) and studies of ecological characteristics of infectious disease vectors for efficient management thereof" in the research program titled "Infectious disease crisis response technology development (Government-wide R&D Fund project for infectious disease research)" by the Industry Foundation of Chonnam National University under management of the Korea Health Industry Development Institute, from 01 August 2018 to 31 December 2020.

This application claims priority to and the benefit of Korean Patent Application No. 10-2019-0009813 filed in the Korean Intellectual Property Office on 25 January 2019,

The present invention relates to general-purpose primer sets for detecting flaviviruses and uses thereof.

### Background Art

Dengue virus, yellow fever virus, Japanese encephalitis virus, West Nile virus, and Zika virus all belong to the genus Flavivirus and the family Flaviviridae, and the diseases therefrom are spread between an animal and a person and between a person and a person by disease-transmitting mosquitoes living inside and outside Korea, such as *Aedes albopictus, Aedes aegypti, Culex pipiens, Culex tritaeniorhynchus,* and *Aedes vexans.*

Dengue fever, caused by dengue virus, is a very painful disease, which occurs in more than 100 countries around the world, and about 2.5 billion people, 40% of the world's population, are exposed to the dengue infection. This disease is mainly distributed in the tropical and subtropical regions and is widely spread up to 35 degrees north/south latitude based on the equator. Korea is not an endemic area for dengue fever, but the number of cases reported in Korea after being infected overseas every year is steadily increasing from 6 cases in 2001 to 313 cases in 2016. There have been reported four genotypes of dengue virus (Type 1, Type 2, Type 3, and Type 4), and it has been known that early treatment in the event of an infection brings a mortality rate of 1% but the mortality rate increases to 20% when the infection develops to severe diseases and causes hemorrhagic fever symptoms, and thus, early diagnosis is a very important. A method of performing a simple test by using a specific antibody after symptom onset and recovery or a method of extracting viral RNA from the blood at the early of symptoms to perform an early viral diagnosis through PCR is used.

Yellow fever is a type of acute viral hemorrhagic fever prevalent in Africa, South America, and North America. Yellow fever virus is known as a causative pathogen, and *Aedes aegypti* has been known to be a major disease vector. There is fever, general pain, vomiting, or the like after the latent period of 3 to 6 days, and shock and coma symptoms occur within 7 to 10 days if appropriate measures are not taken after the onset of symptoms. If jaundice occurs later, the mortality rate is 20 to 50%. As for yellow fever, a method of detecting viral antigens by using antigen/antibody responses through the isolation of viruses from the blood or biopsy and a method of diagnosing the presence or absence of viruses through RT-PCR after the extraction of viral RNA from a sample are mainly used.

Japanese encephalitis is caused by Japanese encephalitis virus, and *Culex tritaeniorhynchus* and *Culex pipiens* are known to be main vectors therefor. Japanese encephalitis is a mosquito-borne viral disease naturalized in Korea as of 2018. Every year, the Korea Centers for Disease Control and Prevention and the Regional Center for Vector Surveillance in Climate Change collect *Culex tritaeniorhynchus,* and then issue a Japanese encephalitis watch across the country in the event of detection of *Culex tritaeniorhynchus* and a Japanese encephalitis warning across the country when the average number of individuals collected per day is 500 or more and the amount of collection exceeds 50% of the total mosquito collection, thereby contributing to the protection of the public health. Japanese encephalitis is mainly found in Asia on the Pacific coast, and more than 1.7 billion people in more than 10 countries are at risk of infection. Japanese encephalitis is endemic in Malaysia and Indonesia and thus continues to plague the inhabitants. Japanese encephalitis is prevalently spread in China, Japan, Korea, and the like at the end of hot and humid summer, and especially, between August and November when mosquitoes are active by Korean standards. Japanese encephalitis, like typical viral diseases, has no special treatment, so prevention is the best. However, fortunately, a vaccine therefor has been developed, and in cases of Koreans, the incidence of the disease is remarkably low due to the vaccination at birth and during infancy. Also for Japanese encephalitis diagnosis, viral diagnosis is made by using antibodies from the blood or by molecular diagnosis using RT-PCR after extraction of viral RNA.

West Nile fever, caused by West Nile virus, was announced as a designated infectious disease in July 2007 and as a fourth group legal infectious disease in 2011 in Korea. In Korea, the West Nile case was first reported around 2012, and as a result of epidemiological investigation, the case was found to develop the disease in Korea after residing in Guinea, Africa. West Nile is a disease that is retained in birds and then transmitted via mosquito vectors to humans and horses, and is prevalent in Africa, Middle East, and Europe. Since West Nile fever is prevalent on a large scale in the eastern part of the United States, West Nile fever is highly likely to flow into Korea, which has many exchanges with the United States, via humans or imported birds. Also for West Nile virus, viral diagnosis can be made by a culture method of isolating virus from a sample and culturing and identifying the virus, an antigen/antibody method using an antibody, or RT-PCR-based molecular diagnostics of detecting a virus-specific gene after nucleic acid extraction.

Zika virus, like yellow fever virus and dengue virus, is transmitted via mosquitoes, and it has been reported that Zika virus may be transmitted via *Aedes aegypti* and *Aedes albopictus.* Since *Aedes albopictus* live in Korea, Zika virus is considered as one of the main vector-borne diseases that can be naturalized in Korea. Zika virus causes a minor Zika fever, but it has been reported that a pregnant woman, when infected, may give birth to a baby with microcephaly, and it has been reported that Zika virus may be transmitted through blood transfusion and sexual contact, and thus extreme concern about Zika virus is spreading all over the world. It has not been long since the Zika virus has been identified as a problematic disease, and thus rather than rapid diagnostics using antibodies, many molecular diagnostics where viral RNA is extracted and Zika virus-specific genes are detected by RT-PCR have been developed.

Since these major pathogens belonging to the genus Flavivirus are spread via mosquitoes, the Centers for Disease Control and Prevention and related institutions around the world are conducting bio-surveillance tasks of continuously surveilling whether new viruses flow into a corresponding region by continuously collecting mosquitoes in the region every year, extracting viral RNA, and performing PCR-based molecular diagnosis. In Korea, quarantine agencies, the Regional Center for Vector Surveillance in Climate Change, the local government Research Institute of Public Health and Environment, and the like are executing related tasks, and are continuously observing the domestic influx of dengue virus, yellow fever virus, Japanese encephalitis virus, West Nile virus, and Zika virus by using RT-PCR and real-time PCR. In Korea, for such public health tasks, a standard test method for detecting viruses from disease-transmitting mosquitoes was constructed, and this method was created on the basis of a thesis reported in 2010 by Yang et al. This paper presented a method for detecting 212bp by using SYBR Green I-based real-time PCR and FL-F1, FL-R3, and FL-R4 primer sets that detect the NS5 gene sequence common to flaviviruses, and presented a method for determining the type of virus through melt curve analysis. Yang et al. Journal of Virological Methods (2010) 168 (1-2) 147-151 describes screening or mosquitoes using Sybr Green I-based real-time RT-PCR with group specific primers for detection of Flavivirus and Alphavirus in Taiwan.

In 2017, Vina-Rodriguez et al. developed a method for detecting new viruses by combining RT-qPCR and a microarray technique to detect new flaviviruses, and this method presented a method of detecting viruses from a sample by using primer sets that detect the NS5 gene sequence possessed by the flavivirus, like in Yang, and then analyzing a PCR product using a microarray. Vina-Rodriguez et al BioMed Research International (2017) 2017 1-12 teaches a novel Pan-Flavivirus Detection and Identification Assay Based on RT-PCR and Microarray.

Studies have been reported to detect various types of viruses from mosquitoes, but the two papers did not resolve false negatives and false positives, and in particular, Yang's method, which is adopted as a standard test method in Korea, dramatically degrades the sensitivity of molecular diagnosis due to structural problems of the primers used for the test, and has a strong false-positive response due to the primer dimerization problem, and therefore, additional tests for re-identifying viral infections need to be carried out. Therefore, there is a need to develop a detection method for dengue virus, yellow fever virus, Japanese encephalitis virus, West Nile virus, and Zika virus, suitable for bio-surveillance.

### Detailed Description of the Invention

### Technical Problem

The present inventors endeavored to develop general-purpose primers capable of promptly and accurately detecting various types of flaviviruses. As a result, the present inventors designed a pair set of primers having optimal amplification efficiency and specificity by aligning the nucleotide sequences of dengue viruses, Japanese encephalitis viruses, West Nile viruses, yellow fever viruses, and Zika viruses, selecting the conserved region, and then performing secondary structure analysis and sequence modification of primers, and established that the use of the primer set can early detect the influx of flaviviruses and the infection with flaviviruses with high sensitivity, and thus completed the present invention.

Accordingly, an aspect of the present invention is to provide a general-purpose primer set for detecting a flavivirus.

Another aspect of the present invention is to provide a kit for diagnosing a flavivirus, the kit including a general-purpose primer set for flavivirus detection.

Still another aspect of the present invention is to provide a method for diagnosing a flavivirus.

### Technical Solution

The present invention is directed to a general-purpose primer set for detecting a flavivirus, to a kit for diagnosing a flavivirus, the kit including the primer set, and to a method for diagnosing a flavivirus by using the same, wherein the use of the primer set of the present invention can detect various types of flaviviruses from a sample with high sensitivity.

Hereinafter, the present invention will be described in more detail.

In accordance with an aspect of the present invention, there is provided a general-purpose primer set for detecting a flavivirus, the primer set including a primer consist of the nucleotide sequence of SEQ ID NO: 1 and a primer consist of the nucleotide sequence of SEQ ID NO: 2.

As used herein, the term "primer" refers to a short nucleic acid sequence having a free 3'-hydroxyl group, which can form base pairs with a complementary template of a plant nucleic acid and serves as a starting point for template strand replication.

In the present invention, oligonucleotides used as the primers may include a nucleotide analogue, for example, a locked nucleic acid (LNA), a phosphorothioate, an alkyl phosphorothioate, or a peptide nucleic acid, or may include an intercalating agent.

According to an embodiment of the present invention, the general-purpose primers for detecting a flavivirus include a locked nucleic acid (LNA).

The general-purpose primers for detecting a flavivirus can be used in the detection of various type of viruses belonging to the genus Flavivirus.

As used herein, the term "flavivirus" refers to a positive single-stranded RNA virus with a size of 40-50 nm belonging to the genus Flavivirus and the family Flaviviridae, wherein main vectors for the flavivirus are mosquitoes or ticks and the flavivirus causes febrile or encephalitis diseases in infected animals or humans.

Examples of the flavivirus detectable using the general-purpose primers for detecting a flavivirus of the present invention are dengue viruses, Japanese encephalitis viruses, West Nile viruses, yellow fever viruses, and Zika viruses.

In accordance with another aspect of the present invention, there is provided a kit for diagnosing a flavivirus, the kit including a general-purpose primer set for flavivirus detection, the primer set being composed of a set of primers of SEQ ID NO: 1 and SEQ ID NO: 2.

The kit may be used to diagnose or detect a flavivirus from a sample.

The kit may include not only a general-purpose primer set for detecting a flavivirus, the primer set including a primer consist of the nucleotide sequence of SEQ ID NO: 1 and a primer consist of the nucleotide sequence of SEQ ID NO: 2, but also a composition of one or more other components, a solution, or a device, which is suitable for analysis.

The kit may be a composition containing essential components necessary to perform PCR analysis, and the kit may include, in addition to respective primer sets, test tubes or other suitable containers, reaction buffers (pH and magnesium concentration being varied), deoxynucleotides (dNTPs), enzymes such as Taqpolymerase, DNase, RNAse inhibitors, DEPC-water, sterile water, and the like.

The kit is not limited to a specific form, and may be implemented in various forms. For example, the kit may include a set of lyophilized primers contained in a container (e.g., disposable PCR tubes, etc.).

In accordance with another aspect of the present invention, there is provided a method for diagnosing a flavivirus, the method including:
an extracting step of extracting RNA from a sample;
a reverse transcription (RT)-PCR performing step of performing RT-PCR by using the extracted RNA as a template and a primer consist of the nucleotide sequence of SEQ ID NO: 1 and a primer consist of the nucleotide sequences of SEQ ID NO: 2;
a target sequence amplifying step of performing PCR by using the RT-PCR product as a template and a primer consist of the nucleotide sequence of SEQ ID NO: 1 and a primer consist of the nucleotide sequence of SEQ ID NO: 2; and
a detecting step of detecting the amplified product.

In the present invention, the sample may be at least one selected from the group consisting of tissues, blood, saliva, urine, semen, and body fluids, but is not limited thereto, and any sample in which a flavivirus can be detected may be used.

In an embodiment of the present invention, a gene amplification reaction may be performed using a primer pair that binds to cDNA synthesized from total RNA obtained from a target virus in a sample.

In the present invention, the RT-PCR performing step may be conducted by a reaction at 40 to 60°C for 5 to 30 minutes, but may be conducted by an ordinary method that can be understood within the level of skill in the art, but is not particularly limited thereto.

In the present invention, the target sequence amplifying step may be conducted by a reaction at 90 to 97°C for 2 to 15 minutes and an amplification of 30 to 45 cycles of 90 to 97°C for 5 to 30 seconds, 56 to 60°C for 5 seconds to 1 minute, 72°C for 10 seconds to 1 minute, and 80 to 85°C for 2 to 10 seconds, but the target sequence amplifying step may be conducted within the level of skill in the art and thus is not particularly limited thereto.

In the present invention, the RT-PCR performing step and the target sequence amplifying step may be consecutively conducted in a single tube, but are not limited thereto.

In the present invention, the detecting step may be conducted using at least one type selected from the group consisting of capillary electrophoresis, DNA chip, gel electrophoresis, radiometric measurement, fluorescence measurement, and phosphorescence measurement, but is not limited thereto. Any detection method by which the amplified product can be detected may be used.

If an amplification product of 257 bp is obtained in the detecting step, it can be determined that the sample has been infected with a flavivirus, and if the melting peak of the sample is observed at 81°C or higher as a result of the melt curve analysis, it can be determined that the sample has been infected with a flavivirus.

### Advantageous Effects

The present invention is directed to a general-purpose primer set for detecting a flavivirus, to a kit for diagnosing a flavivirus, the kit including the primer set, and to a method for diagnosing a flavivirus, wherein Dengue virus type 1, Dengue virus type 2, Dengue virus type 3, Dengue virus type 4, Japanese encephalitis viruses, West Nile viruses, yellow fever viruses, and Zika viruses can be all detected from a sample by using a pair set of general-purpose primers for detecting a flavivirus. Furthermore, through the reduction of a false positive reaction, which is a chronic problem of a conventional general-purpose primer set for detecting a flavivirus, the influx of flaviviruses and infection with flaviviruses can be effectively detected.

### Brief Description of the Drawings

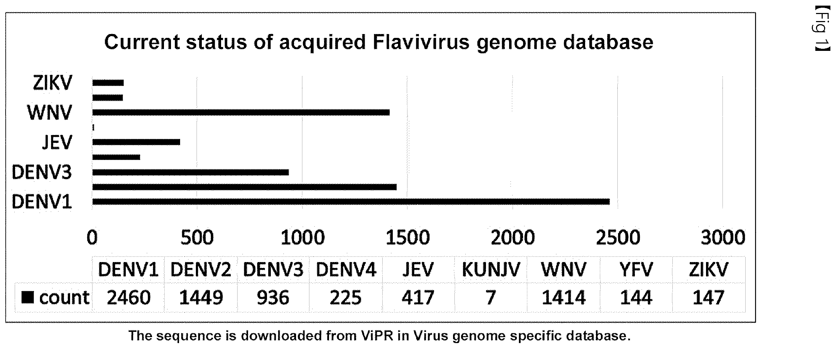

FIG. 1 shows information on flavivirus genomic sequences for use in the designing of a general-purpose primer set for detecting a flavivirus according to an example of the present invention.
FIG. 2A shows a vector constructed for synthesizing an artificial virus sequence for diagnostics verification according to an example of the present invention.
FIG. 2B is a diagram of the synthesis of an artificial virus sequence for diagnostics verification according to an example of the present invention.
FIG. 3A shows the DENV4 detection results using a general-purpose primer set for detecting a flavivirus according to an example of the present invention.
FIG. 3B shows the DENV3 detection results using a general-purpose primer set for detecting a flavivirus according to an example of the present invention.
FIG. 3C shows the DENV2 detection results using a general-purpose primer set for detecting a flavivirus according to an example of the present invention.
FIG. 3D shows the DENV1 detection results using a general-purpose primer set for detecting a flavivirus according to an example of the present invention.
FIG. 3E shows the YFV detection results using a general-purpose primer set for detecting a flavivirus according to an example of the present invention.
FIG. 3F shows the ZIKA detection results using a general-purpose primer set for detecting a flavivirus according to an example of the present invention.
FIG. 3G shows the JEV detection results using a general-purpose primer set for detecting a flavivirus according to an example of the present invention.
FIG. 3H shows the WNV detection results using a general-purpose primer set for detecting a flavivirus according to an example of the present invention.
FIG. 4A shows an amplification curve identified through a RNA panel-based reverse transcription real-time polymerase chain reaction according to an example of the present invention.
FIG. 4B shows a melt curve identified through a diluted RNA panel-based reverse transcription real-time polymerase chain reaction according to an example of the present invention.
FIG. 4C shows amplification and melt curves identified through a RNA panel-based reverse transcription real-time polymerase chain reaction for DENV1 according to an example of the present invention.
FIG. 4D shows amplification and melt curves identified through a RNA panel-based reverse transcription real-time polymerase chain reaction for DENV2 according to an example of the present invention.
FIG. 4E shows amplification and melt curves identified through a RNA panel-based reverse transcription real-time polymerase chain reaction for DENV3 according to an example of the present invention.
FIG. 4F shows amplification and melt curves identified through a RNA panel-based reverse transcription real-time polymerase chain reaction for DENV4 according to an example of the present invention.
FIG. 4G shows amplification and melt curves identified through a RNA panel-based reverse transcription real-time polymerase chain reaction for JEV ANYANG300 according to an example of the present invention.
FIG. 4H shows amplification and melt curves identified through a RNA panel-based reverse transcription real-time polymerase chain reaction for WNV2 according to an example of the present invention.
FIG. 4i shows amplification and melt curves identified through a RNA panel-based reverse transcription real-time polymerase chain reaction for YFV according to an example of the present invention.
FIG. 4J shows amplification and melt curves identified through a RNA panel-based reverse transcription real-time polymerase chain reaction for ZIKA according to an example of the present invention.
FIG. 5A shows the DENV4 detection results using a primer set indicated in the vector-borne flavivirus standard test method according to an example of the present invention.
FIG. 5B shows the DENV3 detection results using a primer set indicated in the vector-borne flavivirus standard test method according to an example of the present invention.
FIG. 5C shows the DENV2 detection results using a primer set indicated in the vector-borne flavivirus standard test method according to an example of the present invention.
FIG. 5D shows the DENV1 detection results using a primer set indicated in the vector-borne flavivirus standard test method according to an example of the present invention.
FIG. 5E shows the YFV detection results using a primer set indicated in the vector-borne flavivirus standard test method according to an example of the present invention.
FIG. 5F shows the ZIKA detection results using a primer set indicated in the vector-borne flavivirus standard test method according to an example of the present invention.
FIG. 5G shows the JEV detection results using a primer set indicated in the vector-borne flavivirus standard test method according to an example of the present invention.
FIG. 5H shows the WNV detection results using a primer set indicated in the vector-borne flavivirus standard test method according to an example of the present invention.
FIG. 6A shows the linearity evaluation results through the Ct value change in reverse transcription real-time polymerase chain reaction performed on DENV1 by using a general-purpose primer set for detecting a flavivirus according to an example of the present invention.
FIG. 6B shows the linearity evaluation results through the Ct value change in reverse transcription real-time polymerase chain reaction performed on DENV3 by using a general-purpose primer set for detecting a flavivirus according to an example of the present invention.
FIG. 6C shows the linearity evaluation results through the Ct value change in reverse transcription real-time polymerase chain reaction performed on DENV2 by using a general-purpose primer set for detecting a flavivirus according to an example of the present invention.
FIG. 6D shows the linearity evaluation results through the Ct value change in reverse transcription real-time polymerase chain reaction performed on DENV4 by using a general-purpose primer set for detecting a flavivirus according to an example of the present invention.
FIG. 6E shows the linearity evaluation results through the Ct value change in reverse transcription real-time polymerase chain reaction performed on JEV by using a general-purpose primer set for detecting a flavivirus according to an example of the present invention.
FIG. 6F shows the linearity evaluation results through the Ct value change in reverse transcription real-time polymerase chain reaction performed on YFV by using a general-purpose primer set for detecting a flavivirus according to an example of the present invention.
FIG. 6G shows the linearity evaluation results through the Ct value change in reverse transcription real-time polymerase chain reaction performed on WNV by using a general-purpose primer set for detecting a flavivirus according to an example of the present invention.
FIG. 6H shows the linearity evaluation results through the Ct value change in reverse transcription real-time polymerase chain reaction performed on ZIKA by using a general-purpose primer set for detecting a flavivirus according to an example of the present invention.
FIG. 7A shows the linearity evaluation results through the Ct value change in reverse transcription real-time polymerase chain reaction performed on DENV1 by using a primer set indicated in the standard test method for vector-borne flavivirus according to an example of the present invention.
FIG. 7B shows the linearity evaluation results through the Ct value change in reverse transcription real-time polymerase chain reaction performed on DENV3 by using a primer set indicated in the standard test method for vector-borne flavivirus according to an example of the present invention.
FIG. 7C shows the linearity evaluation results through the Ct value change in reverse transcription real-time polymerase chain reaction performed on DENV2 by using a primer set indicated in the standard test method for vector-borne flavivirus according to an example of the present invention.
FIG. 7D shows the linearity evaluation results through the Ct value change in reverse transcription real-time polymerase chain reaction performed on DENV4 by using a primer set indicated in the standard test method for vector-borne flavivirus according to an example of the present invention.
FIG. 7E shows the linearity evaluation results through the Ct value change in reverse transcription real-time polymerase chain reaction performed on JEV by using a primer set indicated in the standard test method for vector-borne flavivirus according to an example of the present invention.
FIG. 7F shows the linearity evaluation results through the Ct value change in reverse transcription real-time polymerase chain reaction performed on YFV by using a primer set indicated in the standard test method for vector-borne flavivirus according to an example of the present invention.
FIG. 7G shows the linearity evaluation results through the Ct value change in reverse transcription real-time polymerase chain reaction performed on WNV by using a primer set indicated in the standard test method for vector-borne flavivirus according to an example of the present invention.
FIG. 7H shows the linearity evaluation results through the Ct value change in reverse transcription real-time polymerase chain reaction performed on ZIKA by using a primer set indicated in the standard test method for vector-borne flavivirus according to an example of the present invention.

### Best Mode for Carrying Out the Invention

A general-purpose primer set for detecting a flavivirus, the primer set including a primer consist of the nucleotide sequence of SEQ ID NO: 1 and a primer consist of the nucleotide sequence of SEQ ID NO: 2

### Mode for Carrying Out the Invention

Hereinafter, the present disclosure will be described in more detail by the following examples. However, these examples are used only for illustration, and the scope of the present disclosure is not limited by these examples.

### Example 1: Designing of primers for flavivirus detection

After 1993 species of dengue virus type 1, 1471 species of dengue virus type 2, 943 species of dengue virus type 3, 228 species of dengue virus type 4, 335 species of Japanese encephalitis viruses (types 1, 2, 3, 4, and 5), 1414 species of West Nile viruses (type 1 and type 2), 147 species of yellow fever viruses, and 766 species of Zika viruses (African and Asian types) were subjected to multiple sequence alignment, the most conserved region was selected. Then, through secondary structure analysis and sequence modification of primers, a pair set of primers with optimal amplification efficiency and specificity was prepared as shown in Table 1 below.

**TABLE 1**

| SEQ ID NO: | Name | sequence (5'-3') |
|---|---|---|
| 1 | L2CNU3-F_Pflavi | T+ACA+ACAT+GATGGGVAARAGAGARA ARAA |
| 2 | L2CNU3-R5_Pflavi | C+CR+GCRGTRTCATCRGCRTASA |

| | | |
|---|---|---|
| +A, +T, +G, +C is LNA(locked nucleic acid). | | |

**TABLE 2**

| SEQ ID NO: | Virus | Genbank no. | L2CNU3-F_Pflavi Location |
|---|---|---|---|
| 3 | DENV1 | MF314188 | 8915 - 8943 |
| 4 | DENV2 | MF314189 | 8914 - 8942 |
| 5 | DENV3 | KF954947 | 8883 - 8911 |
| 6 | DENV4 | HQ332173 | 8916 - 8944 |
| 7 | Zika virus | KU501216 | 8917 - 8945 |
| 8 | Yellow fever virus | JN628281 | 8993 - 9021 |
| 9 | Japanese encephalitis virus anyang300 | KF711994 | 8941 - 8969 |
| 10 | West Nile virus | KT359349 | 9006 - 9034 |

**TABLE 3**

| SEQ ID NO: | Virus | Genbank no. | L2CNU3-R5_Pflavi Location |
|---|---|---|---|
| 3 | DENV1 | MF314188 | 9150 - 9171 |
| 4 | DENV2 | MF314189 | 9150 - 9171 |
| 5 | DENV3 | KF954947 | 9118 - 9139 |
| 6 | DENV4 | HQ332173 | 9151 - 9172 |
| 7 | Zika virus | KU501216 | 9152 - 9173 |
| 8 | Yellow fever virus | JN628281 | 9228 - 9249 |
| 9 | Japanese encephalitis virus anyang300 | KF711994 | 9176 - 9197 |
| 10 | West Nile virus | KT359349 | 9241 - 9262 |

### Example 2: Vector construction

Viruses harmful to the human body, such as dengue, Zika, and West Nile viruses, are difficult to culture, and can be handled in only facilities with a biosafety level of 3 or higher due to viral risks. As for the development of molecular diagnostic markers, if viral genome information is known, only a target site can be partially synthesized and used as a template for PCR-based molecular diagnostics. A synthetic gene used in the present invention was newly prepared using a gene synthesis service of Bioneer Co., Ltd., and genetic information was referred to the Genbank DB shown in Table 2.

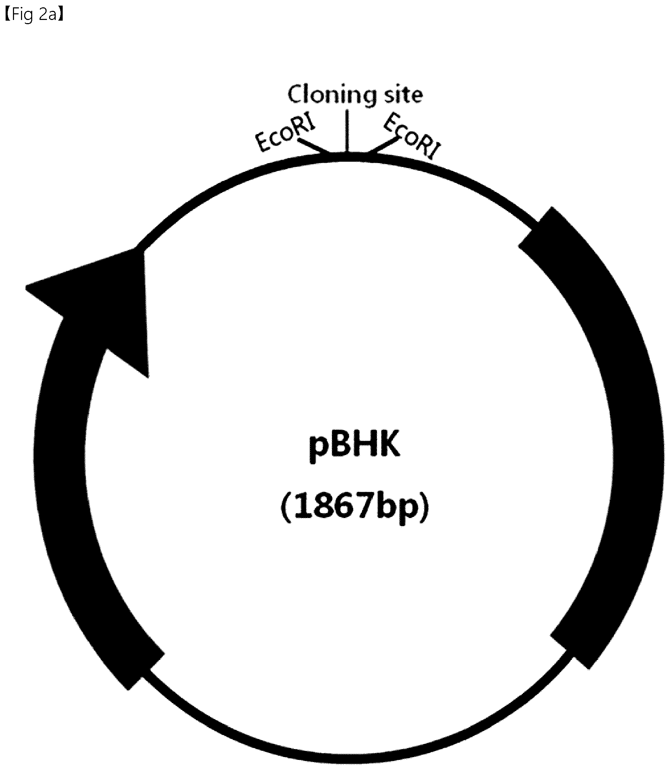

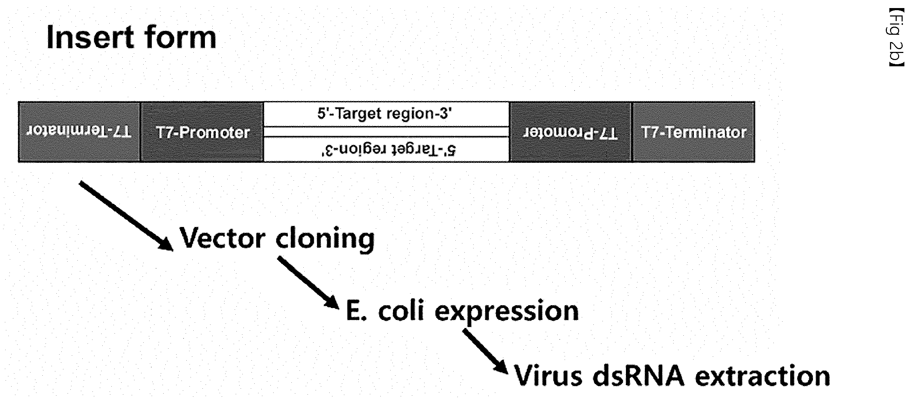

As shown in FIG. 2, the synthetic gene was obtained as dsDNA in the form in which the gene was inserted in the pBHA plasmid vector. However, the immediate use of dsDNA may result in a substantially different virus since the flavivirus is a RNA virus. Therefore, RNA transcription was performed using the synthesized dsDNA as a template, and then the RNA was purified and used as a template for a diagnostic marker.

To this end, the nucleotide sequence of SEQ ID NO: 11 was added to the 5'-end of the synthetic sequence and the nucleotide sequence of SEQ ID NO: 12 was added to the 3'-end thereof.
SEQ ID NO: 11
   TAATACGACTCACTATAGGGTTTGCTAGTTATTGCTCAGCGGTGG
SEQ ID NO: 12
   CCACCGCTGAGCAATAACTAGCTTTCCCTATAGTGAGTCGTATTA

For example, Panel (KT359349|West Nile virus2|2014/hun) is as in the nucleotide sequence of SEQ ID NO: 16. The 1st to 20th parts in the nucleotide sequence of SEQ ID NO: 16 are the sequence of T7 promoter, and when the corresponding sequence is present, T7 RNA polymerase may bind to the T7 promoter to transcribe RNA using DNA as a template. Thereafter, the sensitivity and accuracy of the molecular diagnostic marker were evaluated using the synthesized dsRNA as a template.

### Example 3: Flavivirus detection test

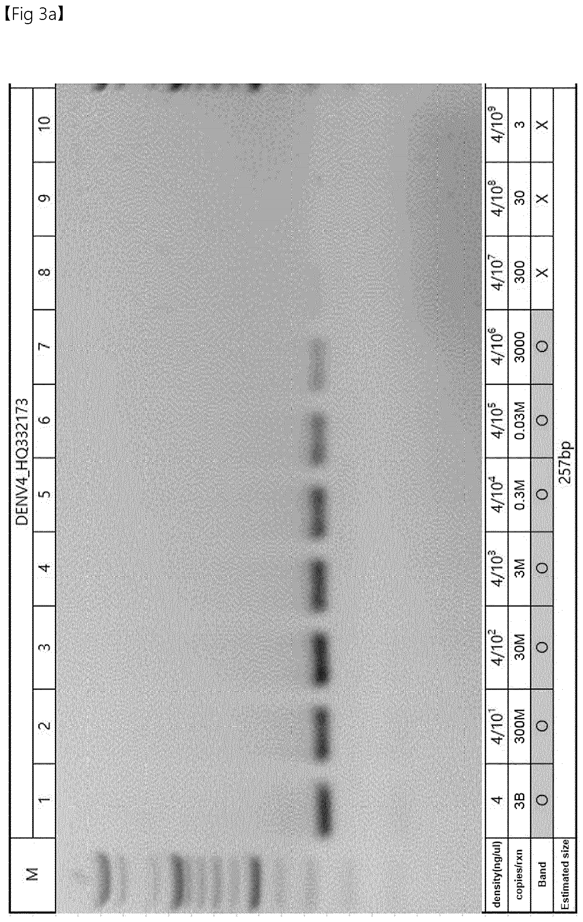

RNA was isolated from a sample, and PCR is performed by 1-step RT-PCR wherein reverse transcription and PCR occur sequentially in the same tube, according to the conditions of Table 4 with the composition of Table 3. The results are shown in FIGS. 3A to 3H and Table 6.

**TABLE 4**

| Element | usage |
|---|---|
| 2x qRT-PCR master mix | 10 ul |
| Primer (10pmole/ul) Fw | 1 ul |
| Primer (10pmole/ul) Rev | 1 ul |
| RNA | 5 ul |
| Molecular biology grade water | 3 ul |

**TABLE 5**

| Step | Temper ature | Time | cycle |
|---|---|---|---|
| RT reaction | 50□ | 15 min | 1 cycle |
| Hot start | 95□ | 10 min | 1 cycle |
| Denature | 95□ | 10 sec | 40 |
| Annealing | 58□ | 15 sec | cycle |
| Extension | 72□ | 15 sec | |
| Detection | 83□ | 5 sec | |
| Dissociatio n | 65□-95□ | | |
| | 0.5□/5sec | | |

**TABLE 6**

| Samp le | Virus | Copies/reaction | Cq | Melting temperature |
|---|---|---|---|---|
| 1 | DENV1 | 3*E9 | 9.74 | 81.5 |
| 2 | DENV1 | 3*E8 | 14.52 | 81.5 |
| 3 | DENV1 | 3*E7 | 17.48 | 81.5 |
| 4 | DENV1 | 3*E6 | 21.24 | 81.5 |
| 5 | DENV1 | 3*E5 | 25.07 | 81.5 |
| 6 | DENV1 | 3*E4 | 28.83 | 81.5 |
| 7 | DENV1 | 3*E3 | 31.85 | 81.5 |
| 8 | DENV1 | 3*E2 | 38.04 | 82 |
| 9 | DENV1 | 3*E1 | No Cq | 74.5 |
| 10 | DENV1 | 3*E0 | No Cq | 67.5 |
| 11 | DENV2 | 3*E9 | 10.28 | 82 |
| 12 | DENV2 | 3*E8 | 15.84 | 82 |
| 13 | DENV2 | 3*E7 | 18.56 | 82 |
| 14 | DENV2 | 3*E6 | 22.23 | 82 |
| 15 | DENV2 | 3*E5 | 26.1 | 82 |
| 16 | DENV2 | 3*E4 | 29.81 | 82.5 |
| 17 | DENV2 | 3*E3 | 33.57 | 82.5 |
| 18 | DENV2 | 3*E2 | 36.49 | 82.5 |
| 19 | DENV2 | 3*E1 | 39.22 | 83.5 |
| 20 | DENV2 | 3*E0 | No Cq | 75 |
| 21 | DENV3 | 3*E9 | 8.54 | 81.5 |
| 22 | DENV3 | 3*E8 | 12.93 | 81.5 |
| 23 | DENV3 | 3*E7 | 16.36 | 81.5 |
| 24 | DENV3 | 3*E6 | 19.98 | 81.5 |
| 25 | DENV3 | 3*E5 | 23.99 | 81.5 |
| 26 | DENV3 | 3*E4 | 27.7 | 81.5 |
| 27 | DENV3 | 3*E3 | 32.02 | 82 |
| 28 | DENV3 | 3*E2 | 35.42 | 82 |
| 29 | DENV3 | 3*E1 | 36.97 | 82.5 |
| 30 | DENV3 | 3*E0 | 39.09 | 85.5 |
| 31 | DENV4 | 3*E9 | 13.69 | 82.5 |
| 32 | DENV4 | 3*E8 | 18.4 | 82.5 |
| 33 | DENV4 | 3*E7 | 20.34 | 82.5 |
| 34 | DENV4 | 3*E6 | 23.97 | 82.5 |
| 35 | DENV4 | 3*E5 | 27.83 | 82.5 |
| 36 | DENV4 | 3*E4 | 31.42 | 82.5 |
| 37 | DENV4 | 3*E3 | 35 | 83 |
| 38 | DENV4 | 3*E2 | 39 | 83 |
| 39 | DENV4 | 3*E1 | No Cq | 65 |
| 40 | DENV4 | 3*E0 | No Cq | 65 |
| 41 | JEV ANYANG | 3*E9 | 10.74 | 83.5 |
| 42 | JEV ANYANG | 3*E8 | 15.33 | 83 |
| 43 | JEV ANYANG | 3*E7 | 18.46 | 83 |
| 44 | JEV ANYANG | 3*E6 | 22.19 | 83.5 |
| 45 | JEV ANYANG | 3*E5 | 26.1 | 83.5 |
| 46 | JEV ANYANG | 3*E4 | 29.9 | 83.5 |
| 47 | JEV ANYANG | 3*E3 | 33.7 | 83.5 |
| 48 | JEV ANYANG | 3*E2 | 38.24 | 84 |
| 49 | JEV ANYANG | 3*E1 | No Cq | 65 |
| 50 | JEV ANYANG | 3*E0 | No Cq | 95 |
| 51 | WNV | 3*E9 | 11.72 | 83.5 |
| 52 | WNV | 3*E8 | 16.62 | 83.5 |
| 53 | WNV | 3*E7 | 19.87 | 83.5 |
| 54 | WNV | 3*E6 | 23.66 | 83.5 |
| 55 | WNV | 3*E5 | 27.53 | 84 |
| 56 | WNV | 3*E4 | 31.35 | 84 |
| 57 | WNV | 3*E3 | 34.66 | 84 |
| 58 | WNV | 3*E2 | 36.56 | 85 |
| 59 | WNV | 3*E1 | No Cq | 65 |
| 60 | WNV | 3*E0 | No Cq | 67.5 |
| 61 | YFV | 3*E9 | 9.48 | 83 |
| 62 | YFV | 3*E8 | 14.12 | 83 |
| 63 | YFV | 3*E7 | 16.99 | 83 |
| 64 | YFV | 3*E6 | 20.78 | 83 |
| 65 | YFV | 3*E5 | 24.55 | 83 |
| 66 | YFV | 3*E4 | 28.19 | 83 |
| 67 | YFV | 3*E3 | 32.14 | 83 |
| 68 | YFV | 3*E2 | 36.35 | 83 |
| 69 | YFV | 3*E1 | No Cq | 67 |
| 70 | YFV | 3*E0 | No Cq | 67.5 |
| 71 | ZIKA | 3*E9 | 10.45 | 83 |
| 72 | ZIKA | 3*E8 | 15.03 | 83 |
| 73 | ZIKA | 3*E7 | 18.22 | 83 |
| 74 | ZIKA | 3*E6 | 22.04 | 83 |
| 75 | ZIKA | 3*E5 | 26.18 | 83.5 |
| 76 | ZIKA | 3*E4 | 30.28 | 83.5 |
| 77 | ZIKA | 3*E3 | 33.96 | 83.5 |
| 78 | ZIKA | 3*E2 | 36.87 | 83.5 |
| 79 | ZIKA | 3*E1 | No Cq | 67.5 |
| 80 | ZIKA | 3*E0 | No Cq | 67 |
| 81 | NTC | NTC | No Cq | 76.5 |
| 82 | NTC | NTC | No Cq | 75.5 |

As can be confirmed in FIGS. 3A to 3H, dengue virus type 1, dengue virus type 2, dengue virus type 3, dengue virus type 4, Japanese encephalitis virus, West Nile virus, yellow fever virus, and Zika virus all could be detected (257 bp). Primer dimers were not observed in low concentrations and no template control. All the types of viruses were detected, and PCR bands were also obviously shown.

As can be confirmed in Table 6, as low as 3 copies of dengue virus type 3 could be detected; as low as 30 copies of dengue virus type 4 and type 2 could be detected; as low as 300 copies of dengue virus type 1, yellow fever virus, Zika virus, Japanese encephalitis virus, and West Nile virus could be detected.

The lowest detection concentration limit of the primer set of the present invention selected through oligonucleotide sequence modification and primer structure optimization for increasing specificity and sensitivity was 3 to 300 copies/rxn, which was significantly improved compared with 3,000,000 copies/rxn, the lowest detection concentration limit of a conventional general-purpose primer set for detecting a flavivirus. Therefore, the primer set of the present invention has no false-positive problem, which is a chronic problem of a conventional general-purpose primer set for detecting a flavivirus.

### Example 4: Comparison with standard test method of Korea Centers for Disease Control and Prevention

### 4-1. Amplification curve and melt curve analysis

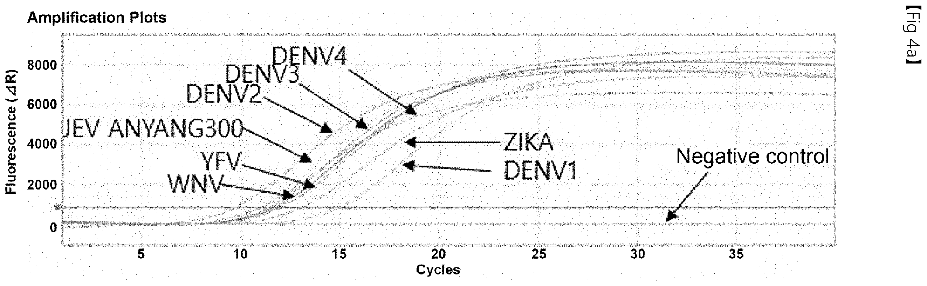

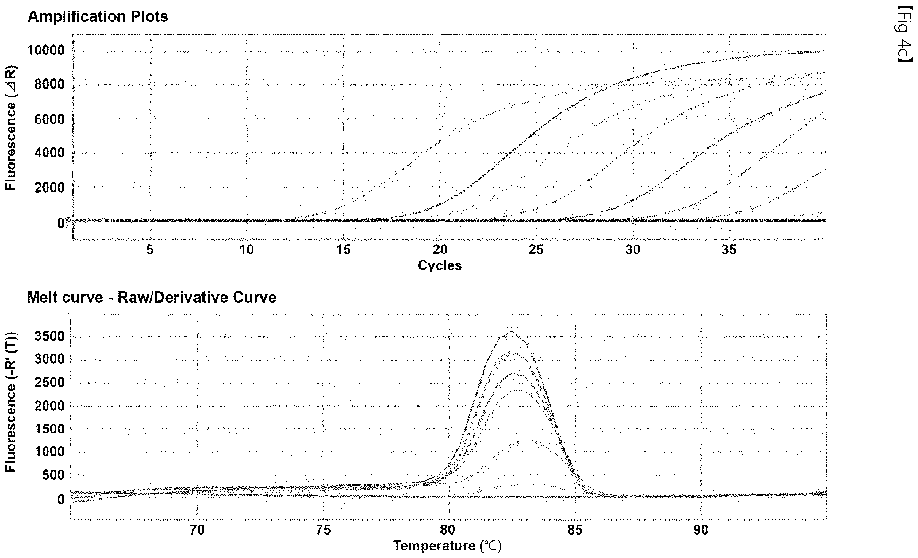

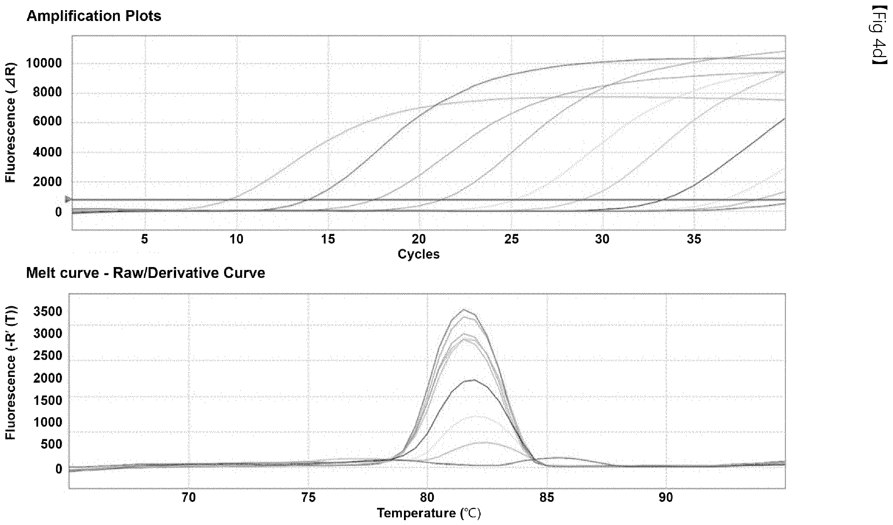

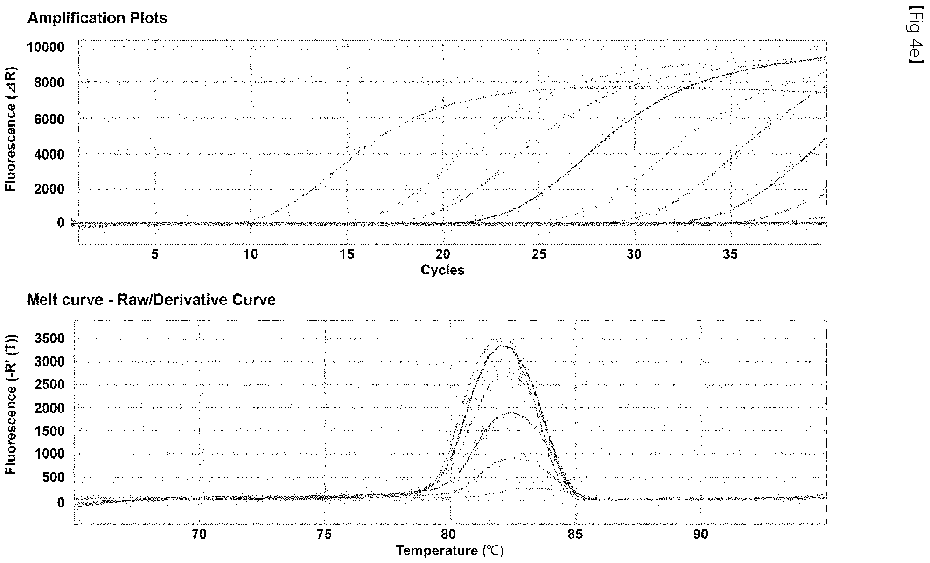

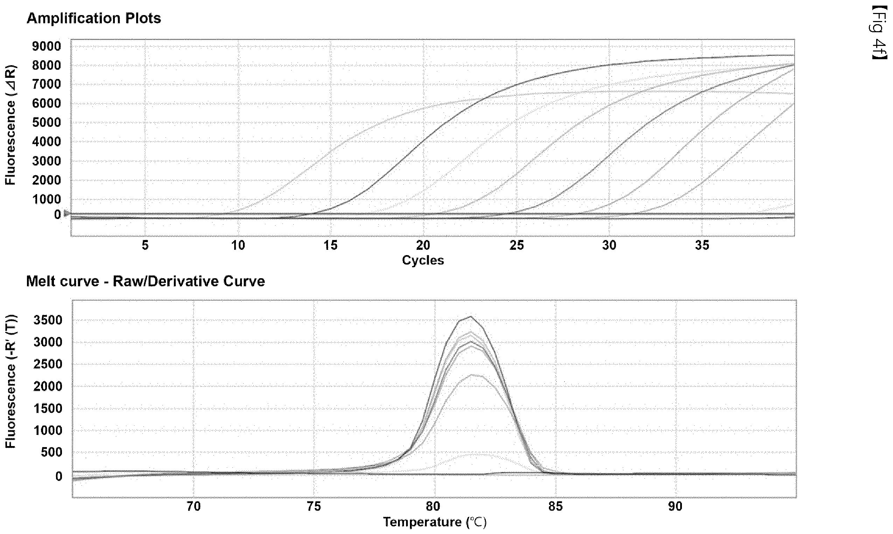

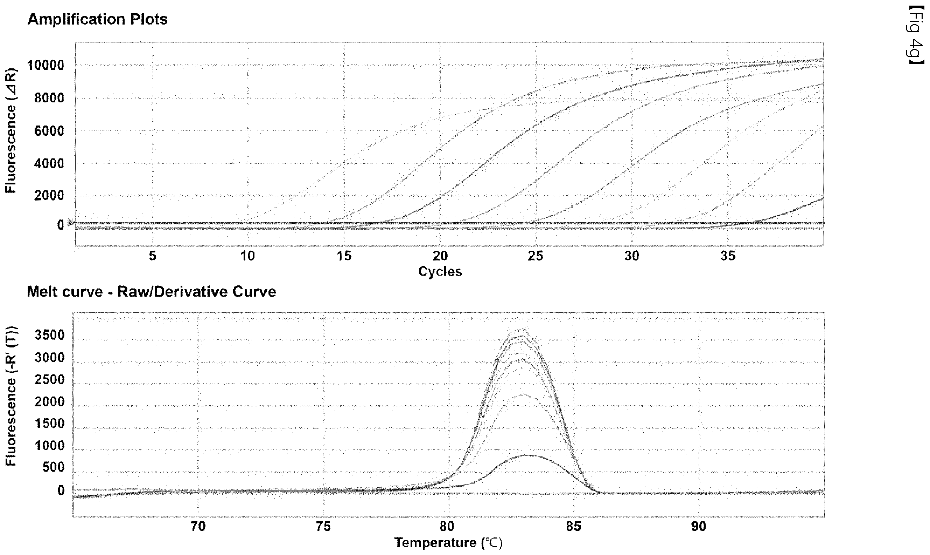

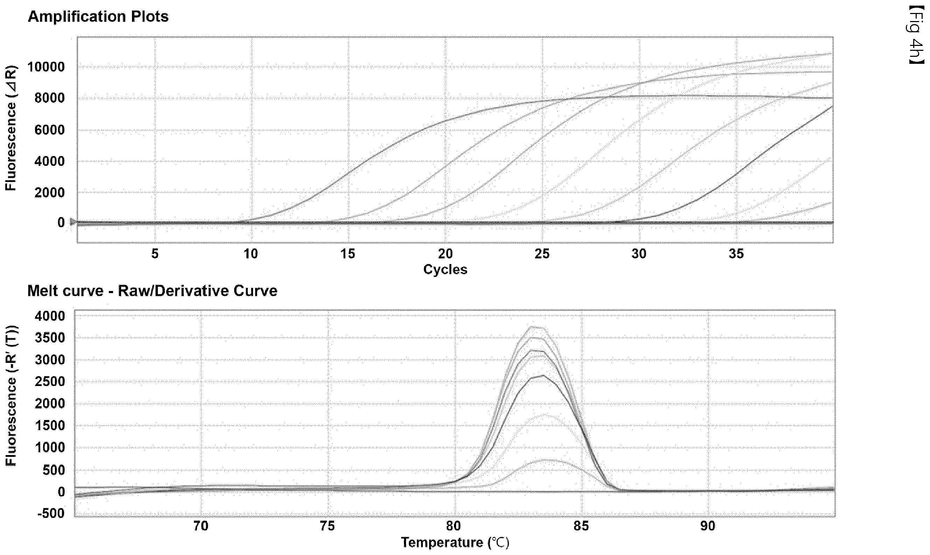

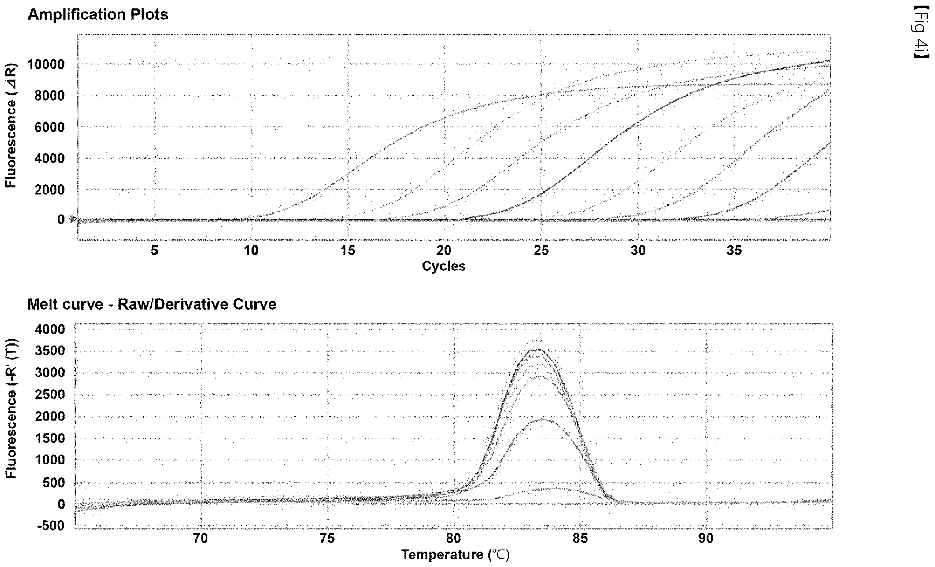

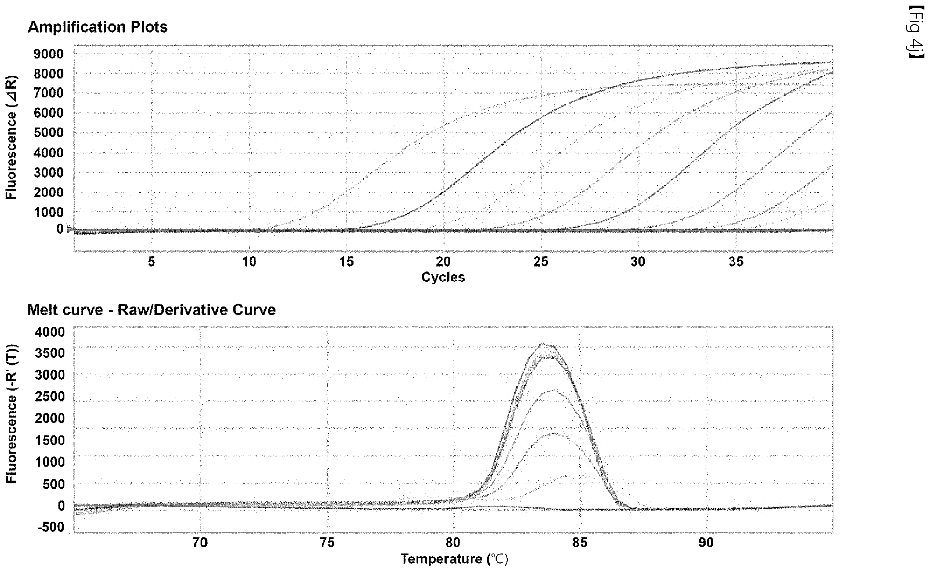

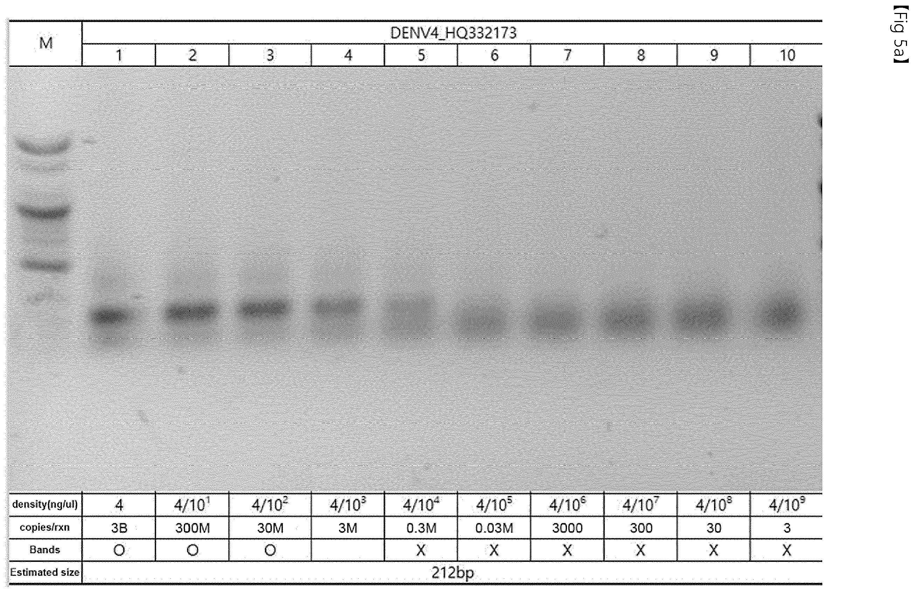

According to the standard test method for vector-borne flavivirus detection, positivity was determined based on the melt curve after real-time PCR was performed using Verso SYBR Green 1-Step RT-qPCR, Thermo Fisher Scientific. Information of primers for pathogen detection is shown in Table 7; the composition of a reagent used is shown in Table 8; the real-time reverse transcription-PCR conditions are shown in Table 9; and the melt curve analysis method is shown in Table 10. In order to distinguish whether the fluorescence signal shown in the amplification plot analysis is a virus-specific product or is a primer dimer or a non-specific product, the melt curve was analyzed under the conditions shown in Table 10, and the results are shown in FIGS. 4A to 4H. In addition, PCR was performed through the method, and then the amplified product was subjected to electrophoresis, and the results are shown in FIGS. 5A to 5H.

**TABLE 7**

| Name | Polarity | Sequence (5' to 3') | SEQ ID NO: |
|---|---|---|---|
| FL-F1 | sense | GCCATATGGTACATGTGGCTGGGAGC | 13 |
| FL-R3 | antisens e | GTKATTCTTGTGTCCCAWCCGGCTGTGT CATC | 14 |
| FL-R4 | antisens e | GTGATGCGRGTGTCCCAGCCRGCKGTGT CATC | 15 |

**TABLE 8**

| Reaction mixture | Volume | Final concentration |
|---|---|---|
| Verso Enzyme Mix | 0.25µℓ | |
| 1-Step QPCR SYBR ROX Mix | 12.5µℓ | 1X |
| RT Enhancer | 1.25µℓ | |
| Forward primer, FL-F1 (1uM) | 1.75µℓ | 70nM |
| Reverse primer, FL-R3 (1uM) | 1.75µℓ | 70nM |
| Reverse primer, FL-R4 (1uM) | 1.75µℓ | 70nM |
| ultra pure water | 0.75µℓ | |
| RNA | 5µℓ | 1pg∼100ng |
| Total reaction volume | 25µℓ | |

**TABLE 9**

| Step | Temperature | Time | Cycle |
|---|---|---|---|
| cDNA synthesis | 50□ | 30min | 1 cycle |
| Inactivation of Reverse | 95□ | 15min | |
| transcriptase | | | |
| Denaturation | 94□ | 15sec | 45cycles |
| Annealing | 58□ | 20sec | |
| Extension* | 72□ | 30sec | |
| * In this step, we set it to read the fluorescent signal. | | | |

**TABLE 10**

| Step | Temperature | Explanation |
|---|---|---|
| Denaturation | 95□ | 30sec |
| Starting temp. | 60□ | 30sec |
| Melting step | ∼ 90□ | Heating 0.5°C by cycle, holding for 10 seconds each cycle and fluorescence analysis |

**TABLE 11**

| borne diseases | melting temperature |
|---|---|
| Japanese encephalitis virus | 83-84□ |
| West Nile virus | 83-84□ |
| Dengue fever virus | 81-82□ |
| Negative control (primer dimer) | 79□ |

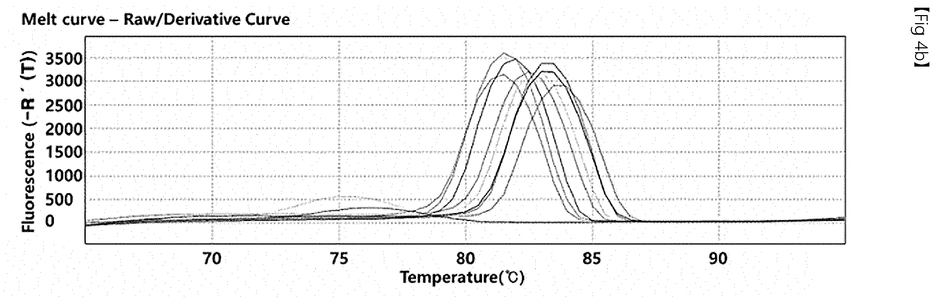

As can be confirmed in FIGS. 4A and 4B, when the primer set is used, neither amplification curve nor melt curve were observed in even the negative control, and thus the number of re-tests can be reduced, the time and cost for additional analysis tests can be saved, and the influx of viruses or infection with viruses can be early detected through a more sensitive detection.

As can be confirmed in FIGS. 5A to 5H, strong primer dimers were observed and bands were not clear in low concentrations of templates and no template control, and WNV was not detected.

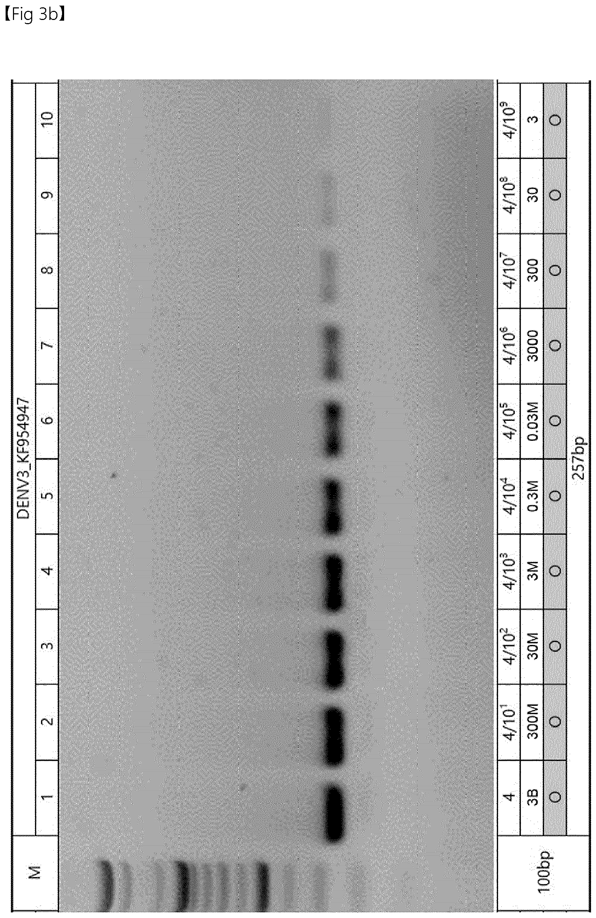

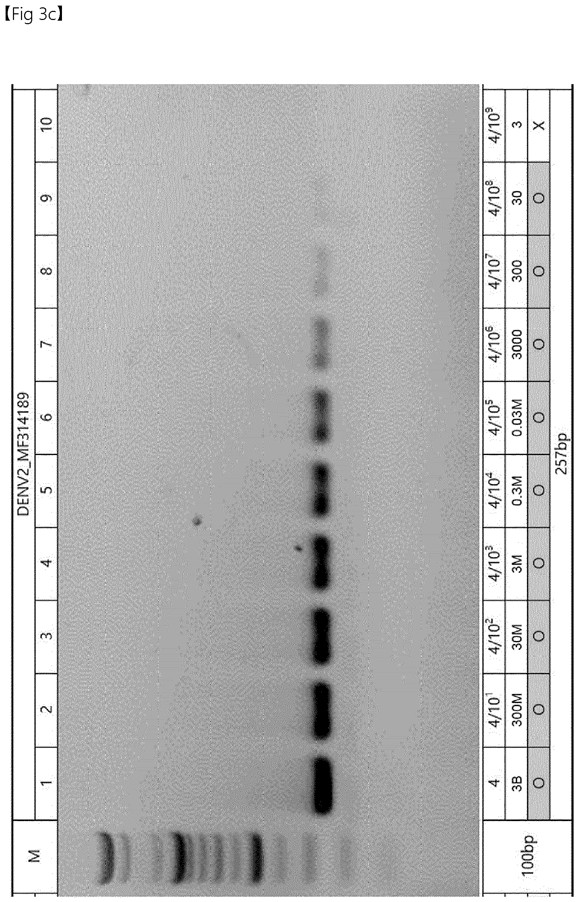

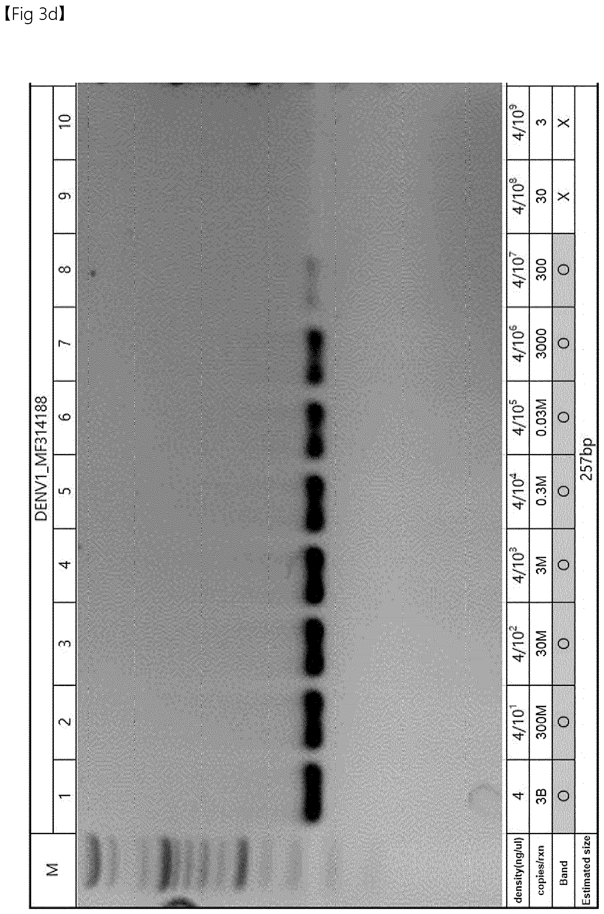

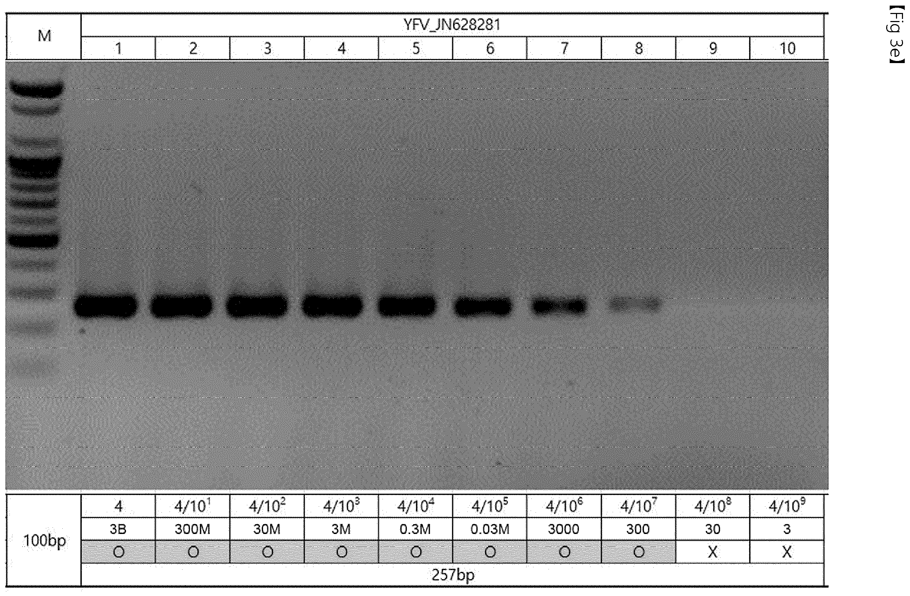

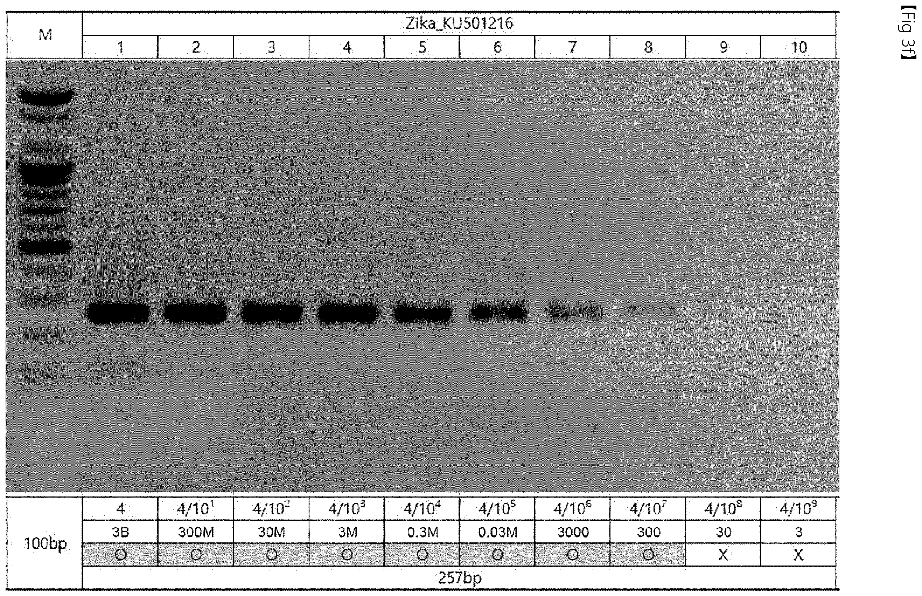

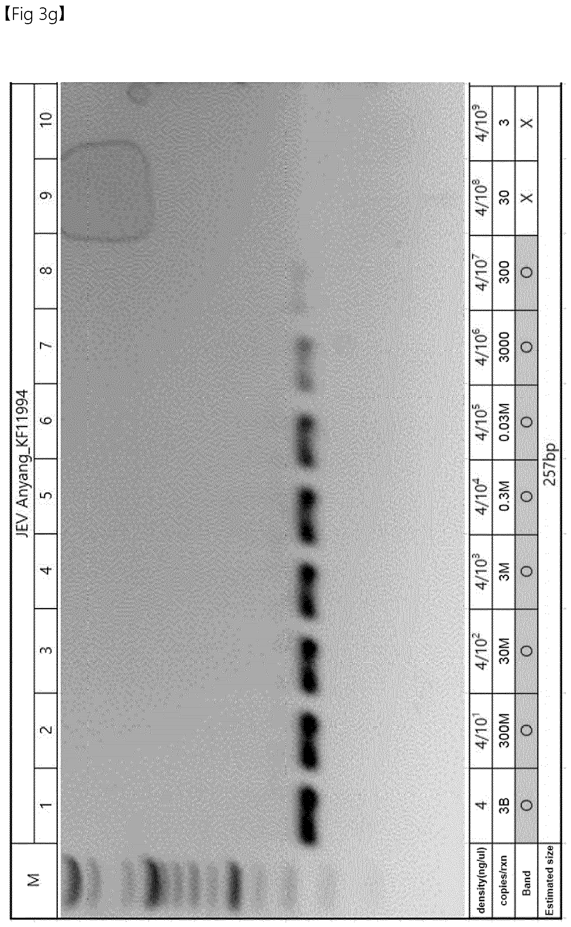

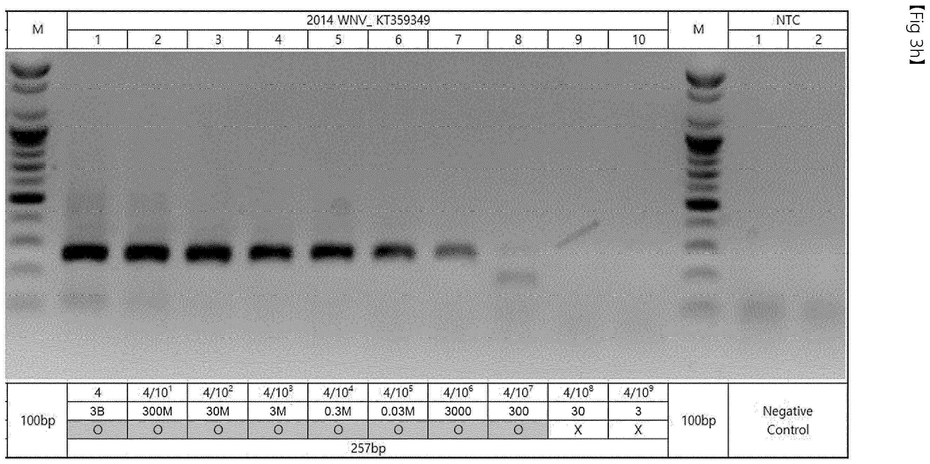

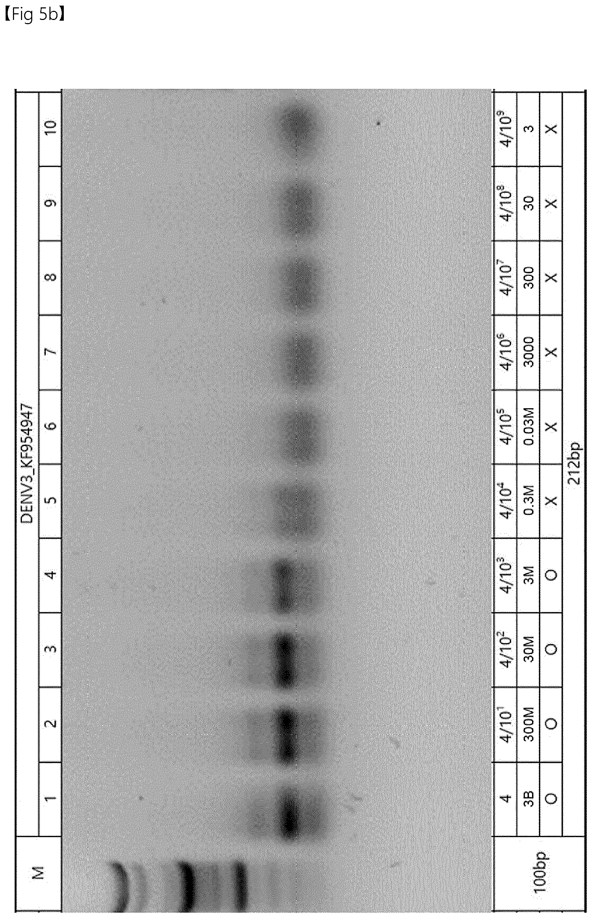

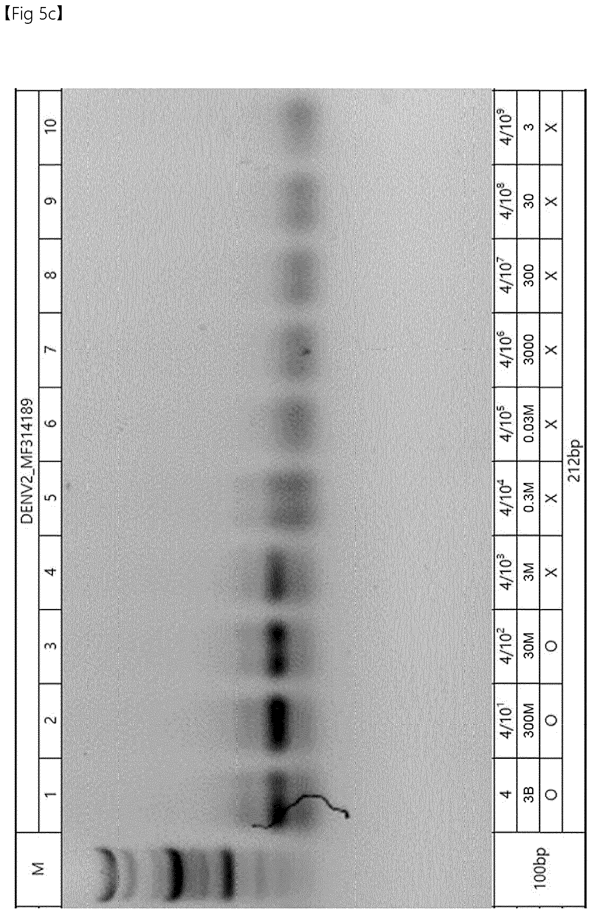

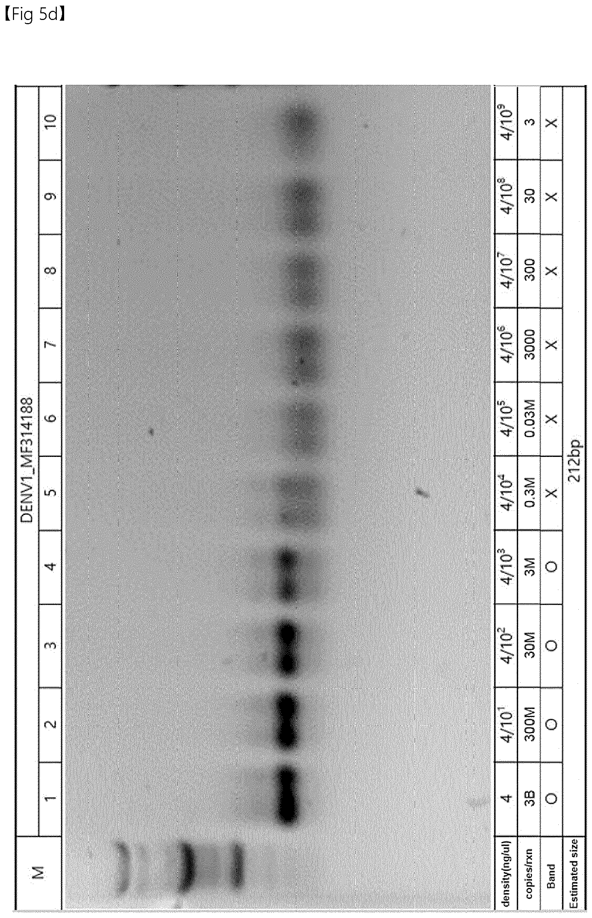

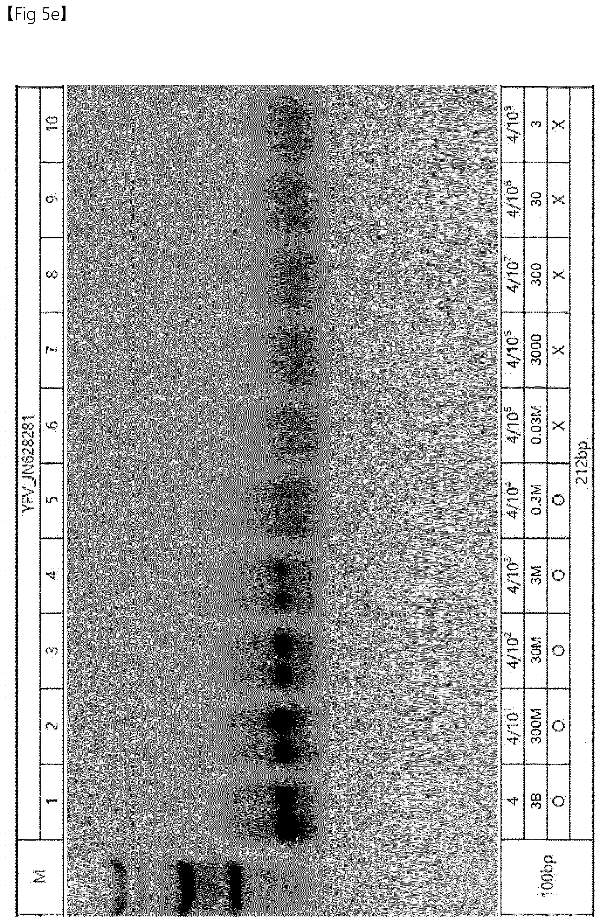

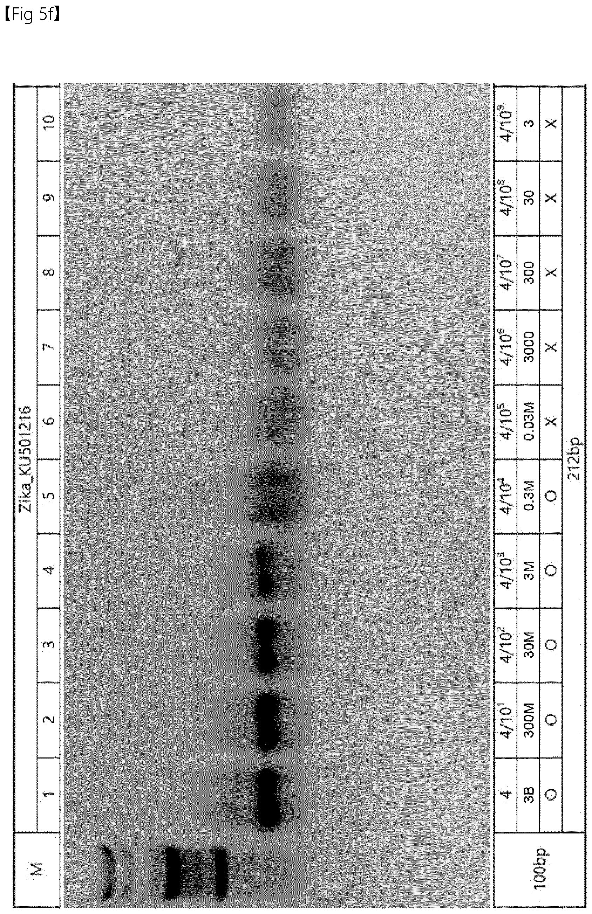

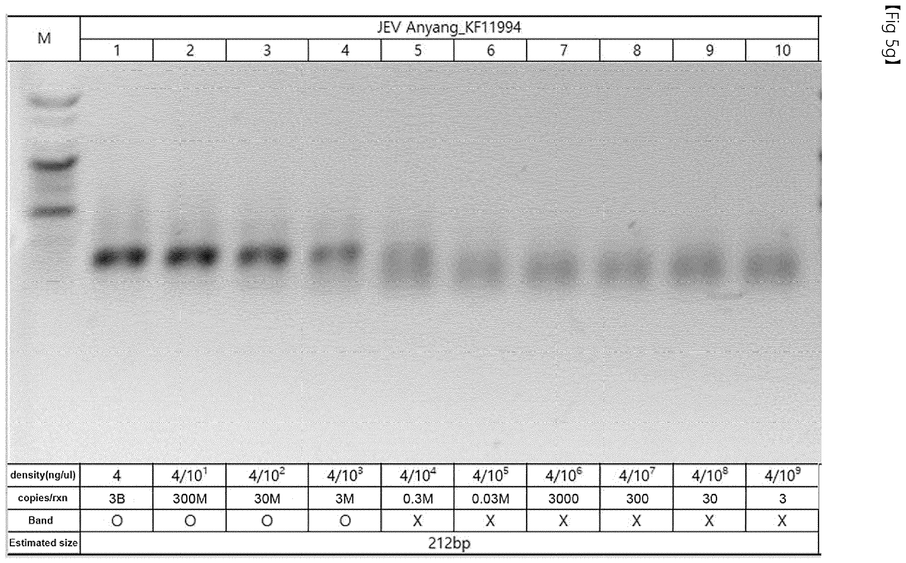

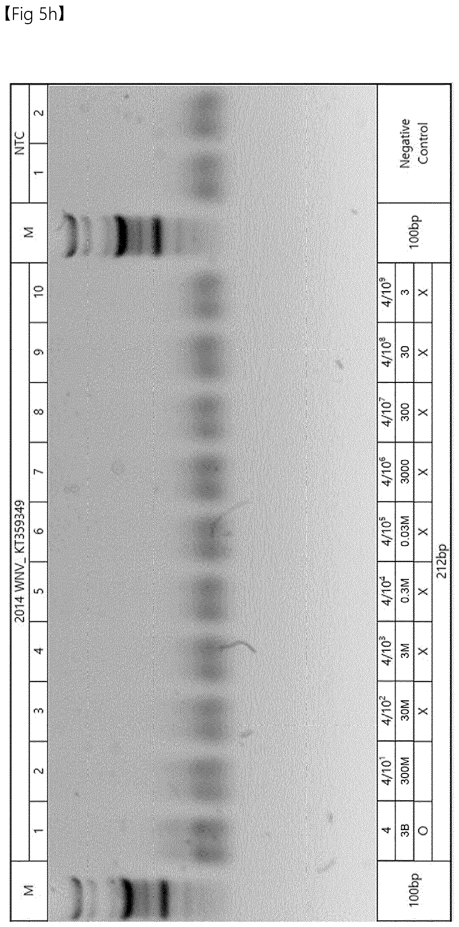

As can be confirmed in FIGS. 3 and 5, the primer set of the present invention in Table 1 showed a sensitivity of up to 1,000,000 times higher than that of the primer set in Table 6 described in the standard test method of the Korea Centers for Disease Control and Prevention.

### 4-2. Linearity evaluation through Ct value change depending on template dilution factor

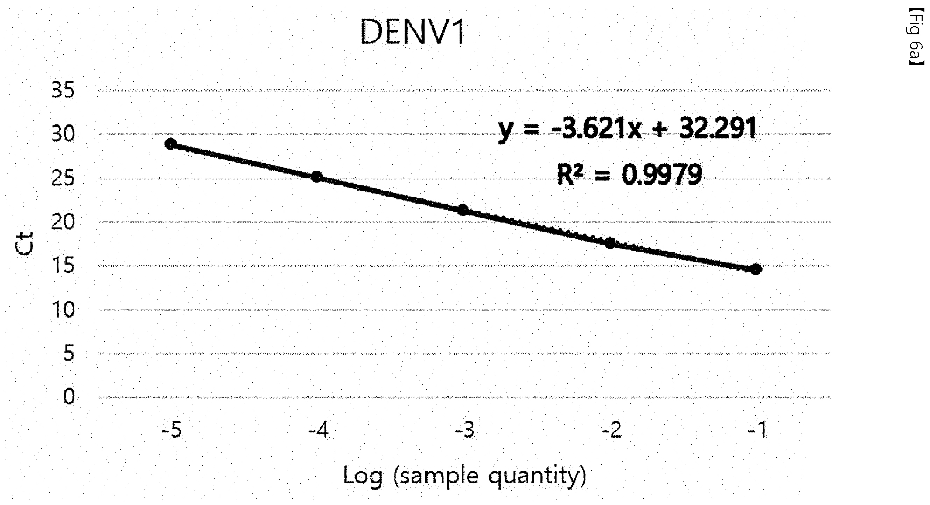

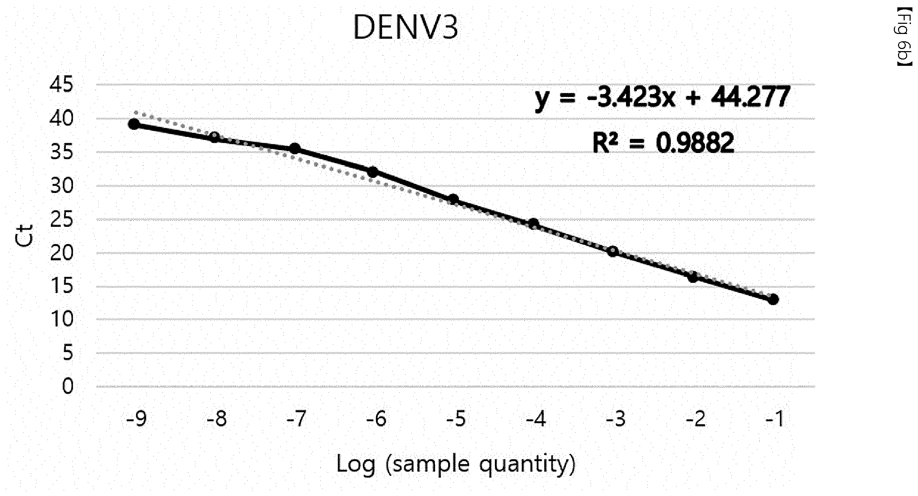

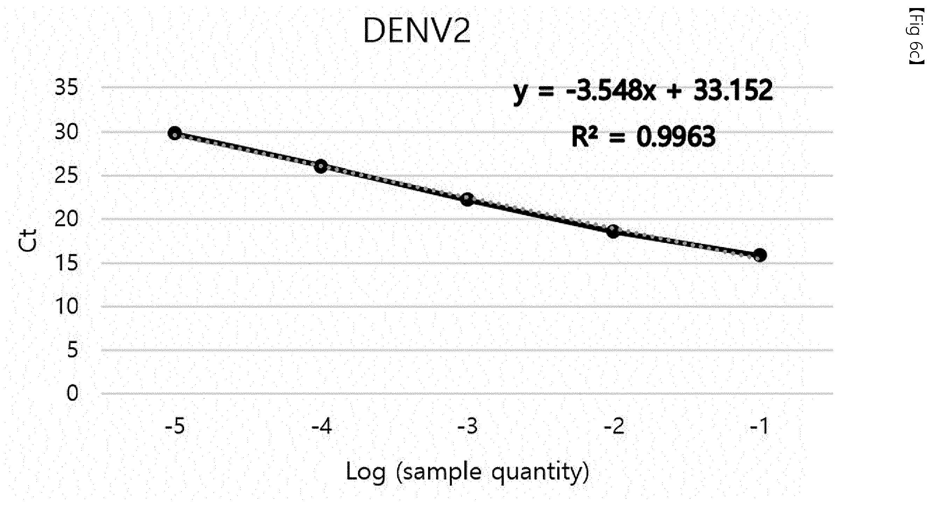

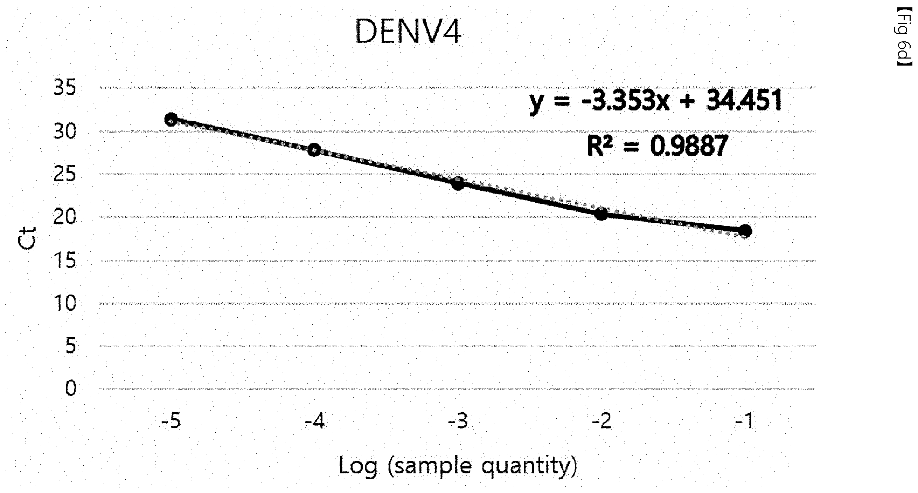

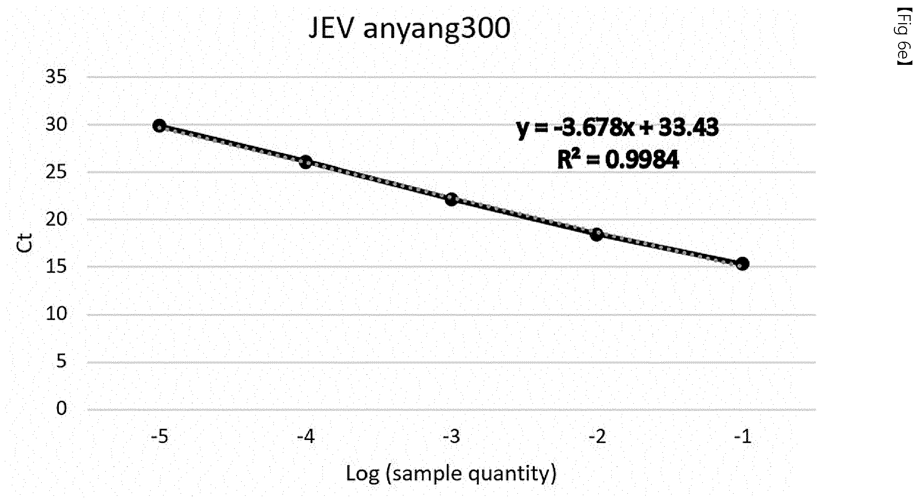

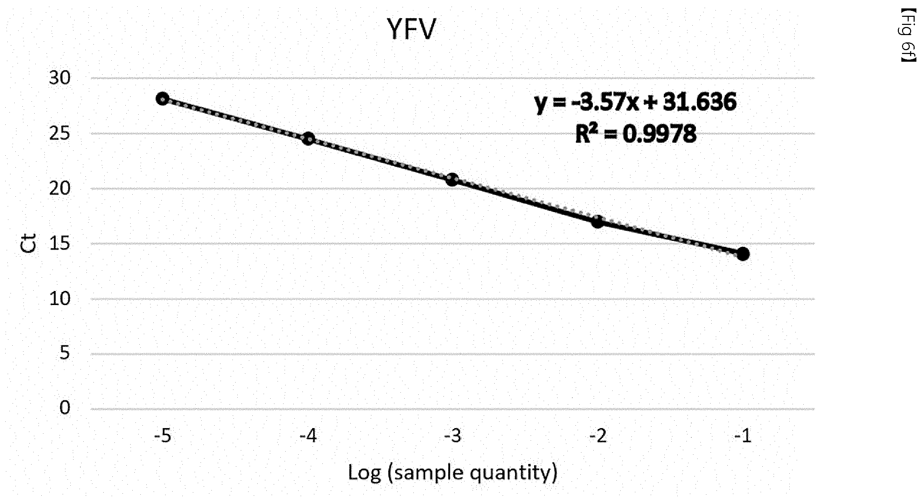

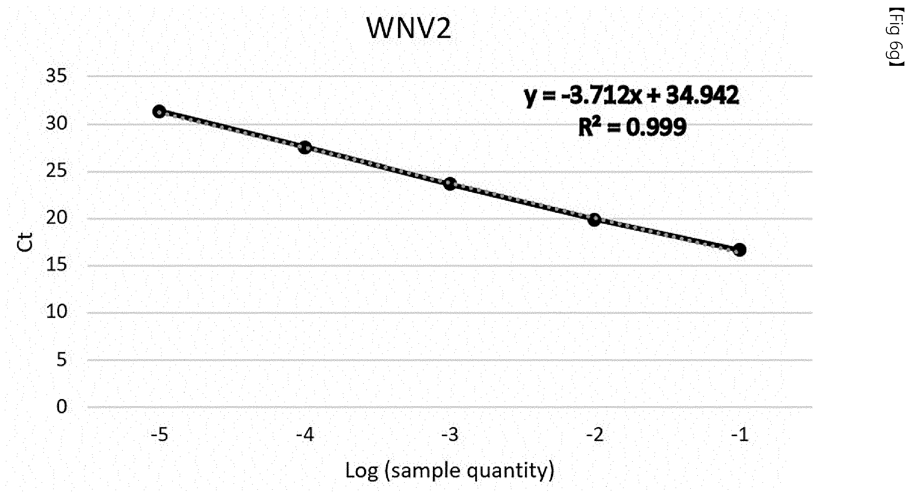

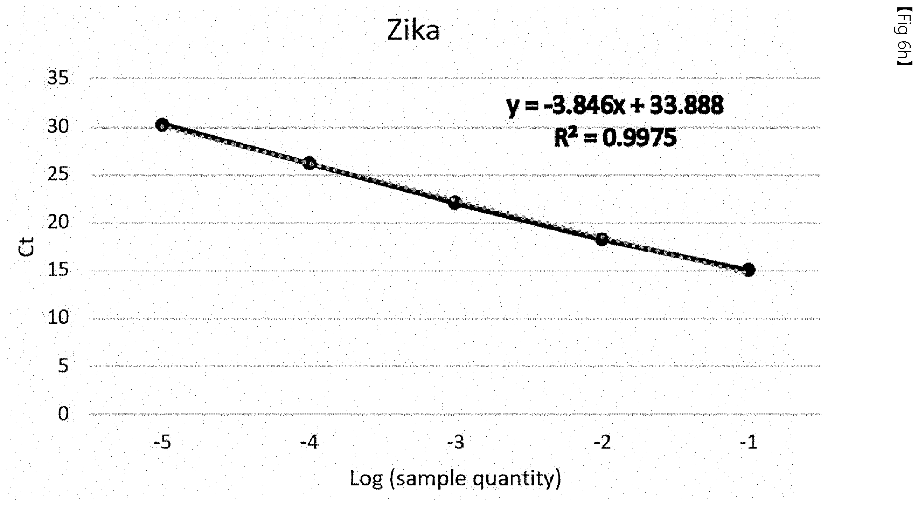

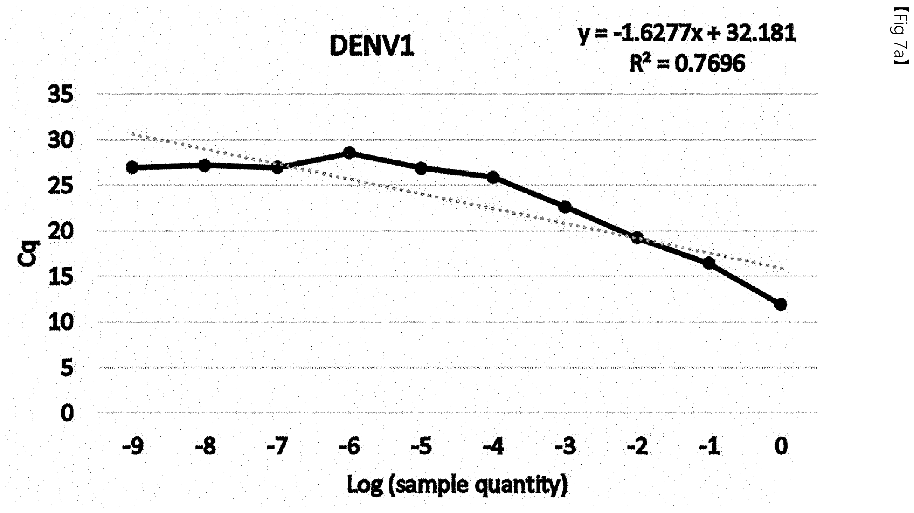

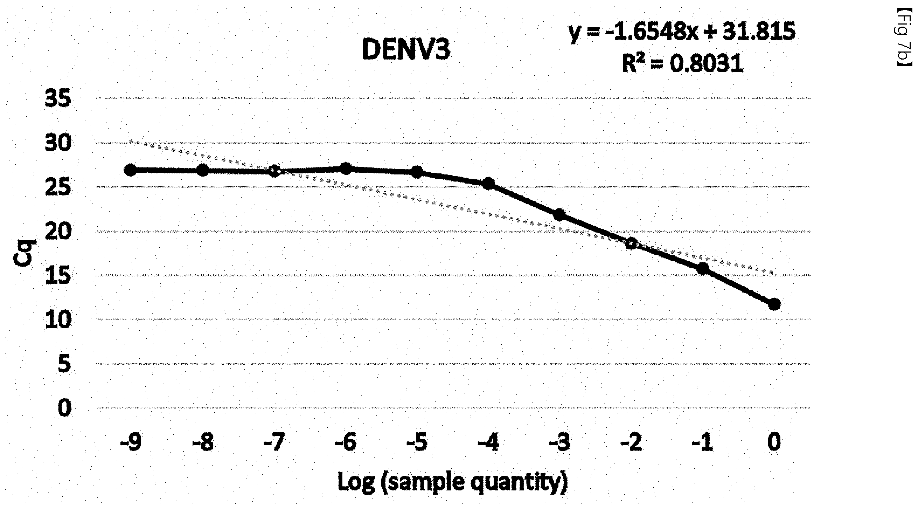

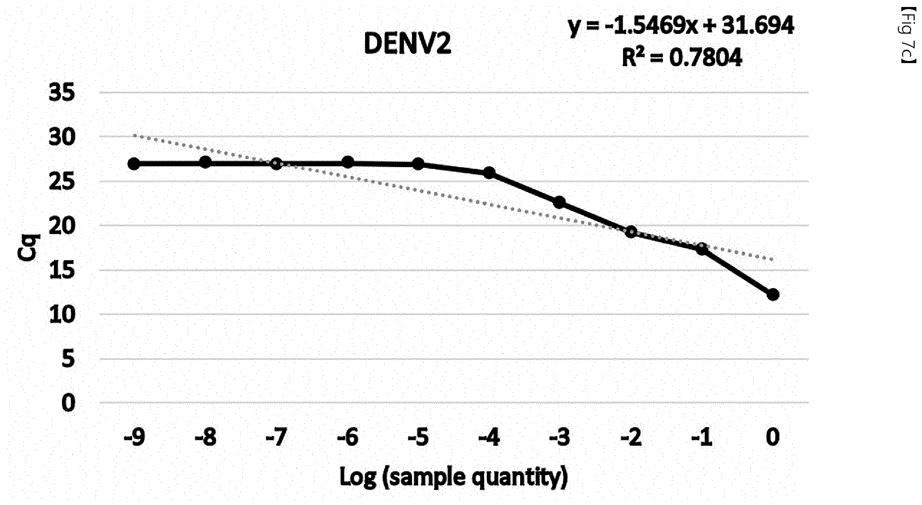

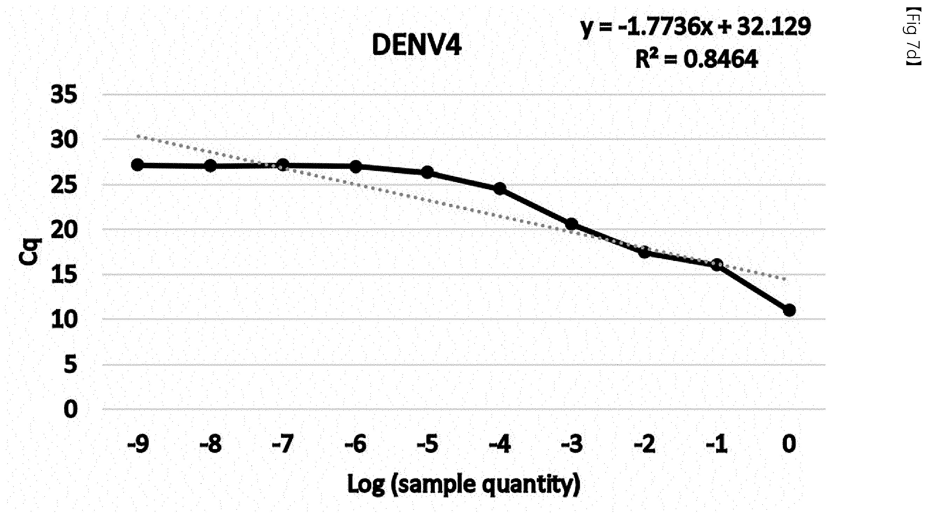

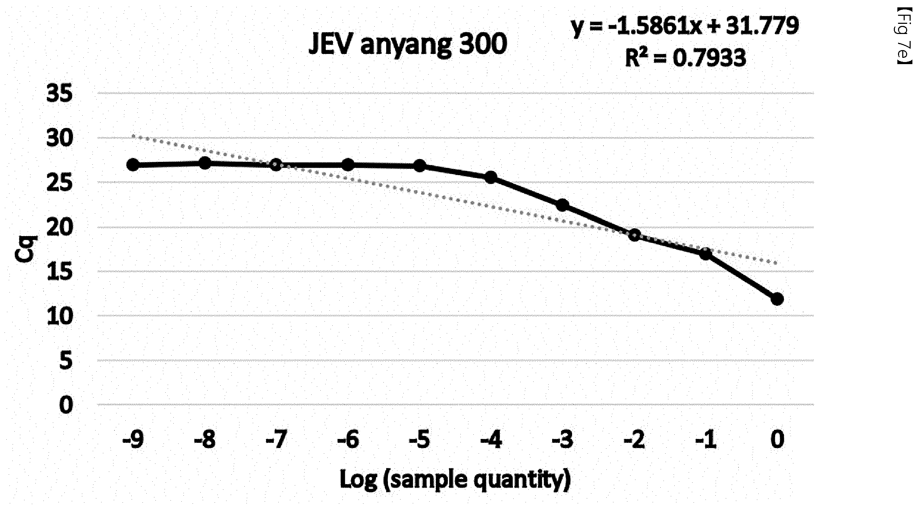

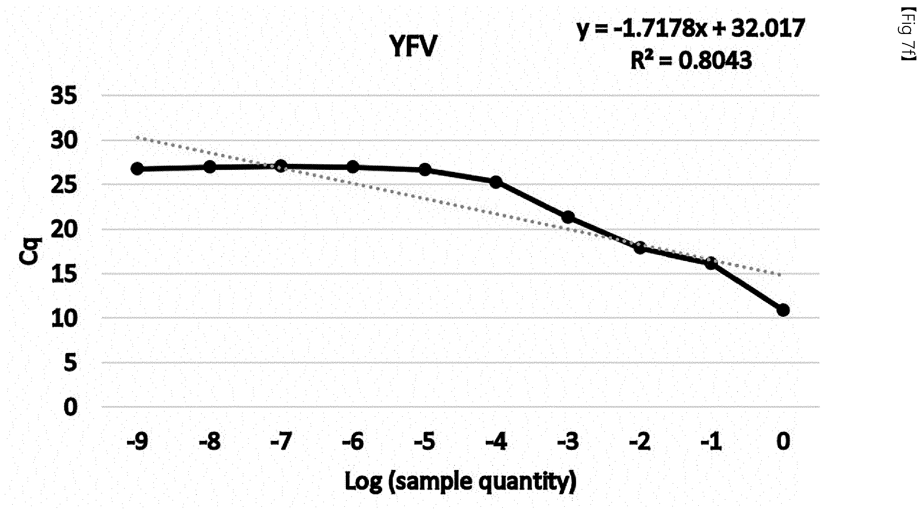

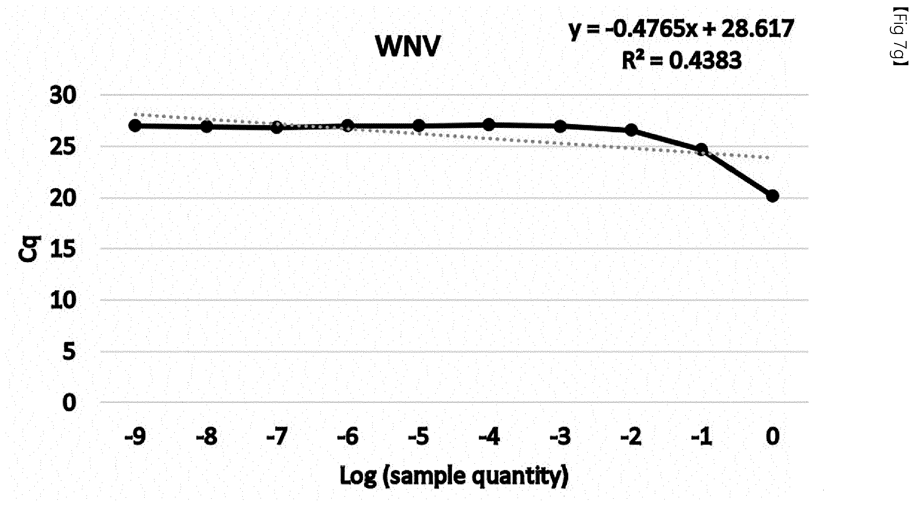

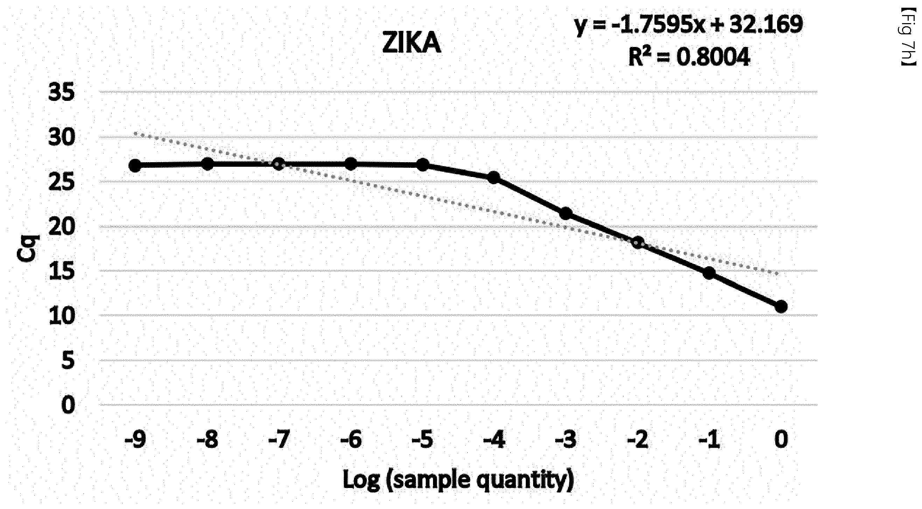

The primer set of the present invention and the primer set described in the standard test method of the Korea Centers for Disease Control and Prevention were evaluated for linearity through the Ct value change depending on the template dilution factor, and the results are shown in FIGS. 6A to 6H and 7A to 7H. The linearity is higher as the R² value is closer to 1. The target gene is difficult to quantify when the linearity is low since the CT value is measured regardless of the amount of the target gene.

As can be confirmed in FIGS. 6A to 6H and 7A to 7H, the R² value was 0.99 on average, close to 1 when the primer set of the present invention was used, but the R² value was 0.75, indicating low linearity when the detection primer set of the Korea Centers for Disease Control and Prevention was used.

### 4-3. Evaluation of PCR efficiency depending on template dilution factor

A PCR efficiency of 90-110% is ideal. The gene detection sensitivity becomes low when the PCR efficiency is much lower than 90%, and the PCR efficiency is higher than 110% when a false-positive reaction occurs or the PCR inhibitor is high in a sample. The PCR efficiency depending on the template dilution factor was evaluated, and shown in Tables 12 and 13.

**TABLE 12**

| | Slope | Efficiency |
|---|---|---|
| DENV1 | -3.621 | 88.87166 |
| DENV2 | -3.54 | 91.35902 |
| DENV3 | -3.423 | 95.94825 |
| DENV4 | -3.353 | 98.71945 |
| JEV ANYANG 300 | -3.678 | 87.0195 |
| WNV | -3.712 | 85.95016 |
| YFV | -3.57 | 90.59524 |
| ZIKA | -3.846 | 81.97444 |

**TABLE 13**

| | Slope | Efficiency |
|---|---|---|
| DENV1 | -1.64479 | 305.4909 |
| DENV2 | -1.56418 | 335.8247 |
| DENV3 | -1.67188 | 296.3962 |
| DENV4 | -1.788 | 262.4801 |
| JEV ANYANG 300 | -1.6023 | 320.8251 |
| WNV | -0.48958 | 10930.24 |
| YFV | -1.73806 | 276.1438 |
| ZIKA | -1.778 | 265.115 |

As can be confirmed in Table 12, the detection method of the present invention showed a PCR efficiency of 80-100%. As can be confirmed in Table 13, the standard test method of the Korea Centers for Disease Control and Prevention showed a PCR efficiency of 265-10930%. These results are made when a high CT value is shown in even a sample with a small amount of templates due to a false-positive reaction by primer dimers.

### Industrial Applicability

The present invention relates to general-purpose primer sets for detecting flaviviruses and uses thereof.

## Claims

1. A general-purpose primer set for detecting a flavivirus, the primer set comprising a primer consist of the nucleotide sequence of SEQ ID NO: 1 and a primer consist of the nucleotide sequence of SEQ ID NO: 2.

2. The primer set of claim 1, wherein the flavivirus is at least one selected from the group consisting of Dengue viruses, Japanese encephalitis viruses, West Nile viruses, Yellow fever viruses, and Zika viruses.

3. A kit for diagnosing a flavivirus, the kit comprising a general-purpose primer set for flavivirus detection, the primer set comprising a primer consist of the nucleotide sequence of SEQ ID NO: 1 and a primer consist of the nucleotide sequence of SEQ ID NO: 2.

4. The kit of claim 3, wherein the flavivirus is at least one selected from the group consisting of Dengue viruses, Japanese encephalitis viruses, West Nile viruses, Yellow fever viruses, and Zika viruses.

5. A method for diagnosing a flavivirus, the method comprising:
an extracting step of extracting RNA from a sample;
a reverse transcription (RT)-PCR performing step of performing RT-PCR by using the extracted RNA as a template and a primer consist of the nucleotide sequence of SEQ ID NO: 1 and a primer consist of the nucleotide sequences of SEQ ID NO: 2;
a target sequence amplifying step of performing PCR by using the RT-PCR product as a template and a primer consist of the nucleotide sequence of SEQ ID NO: 1 and a primer consist of the nucleotide sequence of SEQ ID NO: 2; and
a detecting step of detecting the amplified product.

6. The method of claim 5, wherein the flavivirus is at least one selected from the group consisting of Dengue viruses, Japanese encephalitis viruses, West Nile viruses, Yellow fever viruses, and Zika viruses.

7. The method of claim 5, wherein the sample is at least one selected from the group consisting of tissues, blood, saliva, urine, semen, and body fluids.

## Patentansprüche

1. Universeller Primersatz zum Nachweisen eines Flavivirus, der Primersatz umfassend einen Primer, der aus der Nukleotidsequenz von SEQ ID NO: 1 besteht, und einen Primer, der aus der Nukleotidsequenz von SEQ ID NO: 2 besteht.

2. Primersatz nach Anspruch 1, wobei das Flavivirus mindestens eines ist, das aus der Gruppe ausgewählt ist, die aus Dengue-Viren, Japanische-Enzephalitis-Viren, West-Nil-Viren, Gelbfieber-Viren und Zika-Viren besteht.

3. Kit zum Diagnostizieren eines Flavivirus, wobei das Kit einen universellen Primersatz für einen Flavivirus-Nachweis umfasst, der Primersatz umfassend einen Primer, der aus der Nukleotidsequenz von SEQ ID NO: 1 besteht, und einen Primer, der aus der Nukleotidsequenz von SEQ ID NO: 2 besteht.

4. Kit nach Anspruch 3, wobei das Flavivirus mindestens eines ist, das aus der Gruppe ausgewählt ist, die aus Dengue-Viren, Japanische-Enzephalitis-Viren, West-Nil-Viren, Gelbfieber-Viren und Zika-Viren besteht.

5. Verfahren zum Diagnostizieren eines Flavivirus, wobei das Verfahren Folgendes umfasst:
einen Extraktionsschritt zum Extrahieren von RNA aus einer Probe;
einen Durchführungsschritt für Reverse-Transkription(RT)-PCR zum Durchführen von RT-PCR unter Verwendung der extrahierten RNA als eine Matrize und eines Primers, der aus der Nukleotidsequenz von SEQ ID NO: 1 besteht, und eines Primers, der aus den Nukleotidsequenzen von SEQ ID NO: 2 besteht;
einen Zielsequenz-Amplifikationsschritt zum Durchführen von PCR unter Verwendung des RT-PCR-Produkts als eine Matrize und eines Primers, der aus der Nukleotidsequenz von SEQ ID NO: 1 besteht, und eines Primers, der aus der Nukleotidsequenz von SEQ ID NO: 2 besteht; und
einen Nachweisschritt zum Nachweisen des amplifizierten Produkts.

6. Verfahren nach Anspruch 5, wobei das Flavivirus mindestens eines ist, das aus der Gruppe ausgewählt ist, die aus Dengue-Viren, Japanische-Enzephalitis-Viren, West-Nil-Viren, Gelbfieber-Viren und Zika-Viren besteht.

7. Verfahren nach Anspruch 5, wobei die Probe mindestens eine Probe ist, die aus der Gruppe ausgewählt ist, die aus Geweben, Blut, Speichel, Urin, Samen und Körperflüssigkeiten besteht.

## Revendications

1. Ensemble d'amorces à usage général pour détecter un flavivirus, l'ensemble d'amorces comprenant une amorce constituée de la séquence nucléotidique de SEQ ID NO : 1 et une amorce constituée de la séquence nucléotidique de SEQ ID NO : 2.

2. Ensemble d'amorces selon la revendication 1, dans lequel le flavivirus est au moins un virus choisi dans le groupe constitué des virus de la dengue, des virus de l'encéphalite japonaise, des virus du Nil occidental, des virus de la fièvre jaune et des virus Zika.

3. Kit pour diagnostiquer un flavivirus, le kit comprenant un ensemble d'amorces à usage général pour la détection de flavivirus, l'ensemble d'amorces comprenant une amorce constituée de la séquence nucléotidique de SEQ ID NO : 1 et une amorce constituée de la séquence nucléotidique de SEQ ID NO : 2.

4. Kit selon la revendication 3, dans lequel le flavivirus est au moins un virus choisi dans le groupe constitué par les virus de la dengue, les virus de l'encéphalite japonaise, les virus du Nil occidental, les virus de la fièvre jaune et les virus Zika.

5. Procédé de diagnostic d'un flavivirus, le procédé comprenant :
une étape d'extraction consistant à extraire l'ARN d'un échantillon ;
une étape d'exécution de transcription inverse (RT)-PCR consistant à exécuter une RT-PCR en utilisant l'ARN extrait comme matrice et une amorce constituée de la séquence nucléotidique de SEQ ID NO : 1 et une amorce constituée des séquences nucléotidiques de SEQ ID NO : 2.
une étape d'amplification de séquence cible consistant à effectuer une PCR en utilisant le produit de RT-PCR comme matrice et une amorce constituée de la séquence nucléotidique de SEQ ID NO : 1 et une amorce constituée de la séquence nucléotidique de SEQ ID NO : 2 et
une étape de détection consistant à détecter le produit amplifié.

6. Procédé selon la revendication 5, dans lequel le flavivirus est au moins un virus choisi dans le groupe constitué des virus de la dengue, des virus de l'encéphalite japonaise, des virus du Nil occidental, des virus de la fièvre jaune et des virus Zika.

7. Procédé selon la revendication 5, dans lequel l'échantillon est au moins un échantillon choisi dans le groupe constitué de tissus, de sang, de salive, d'urine, de sperme et de fluides corporels.
